# EUROPEAN PATENT APPLICATION

(11) **EP 0 707 036 A1**
(43) Date of publication of application: **17.04.1996**
(21) Application number: 95116059.7
(22) Date of filing: 11.10.1995
(51) Int. Cl.: C08K 13/02, C07D 251/70

(54) **Flame-retardant polymer compositions and salts of cyanuric acid with triazine compounds useful in the preparation of the compositions**

(30) Priority: 11.10.1994 IT MI942069
(71) Applicant: MONTELL NORTH AMERICA INC., New Castle County Delaware (US)
(72) Inventor: Cipolli, Roberto, I-28100 Novara (IT)
(74) Representative: Zumstein, Fritz, Dr.

(57) **Abstract**

Polymer compositions having high flame-retardant properties comprising:
a) from 90 to 40 parts by weight of a thermoplastic or elastomeric polymer;
b) from 6 to 33 parts by weight of at least one phosphorus compound selected from the group consisting of ammonium and amine phosphates or phosphonates; and
c) from 4 to 27 parts by weight of one or more cyanuric acid salts of derivaties of 2,4,6-triamino-1,3,5-triazine containing two or more triazine rings.

## Description

The present invention relates to a flame-retardant composition comprising a thermoplastic or elastomeric polymer, containing salts of triazine compounds in combination with ammonium or amine phosphates and/or phosphonates. Moreover, the present invention relates to new salts of triazine compounds and to a process for their preparation.

Various solutions for reducing or eliminating the flammability of polymer are known in the art. Some of these solutions are based on the use of antimony, bismuth or arsenic compounds, in combination with partially halogenated and thermally unstable organic compounds, such as chlorinated paraffin waxes.

Other solutions are based on the use of substances capable of causing intumescence. Intumescent formulations generally comprise the polymer and at least three main additives: one essentially comprising a phosphorus compound, whose purpose is to form, when contacted with flame, an impermeable semisolid glass layer, comprising primarily polyphosphoric acid, and to activate the intumescent process, a second additive containing nitrogen, which acts as a foaming agent, and a third additive containing carbon, which acts as a carbon donor for the formation of an insulating cellular carbon layer (char) between polymer and the flame.

Examples of this type of intumescent formulations are described in patents: US 3,810,862 based on melamine, pentaerythritol and ammonium polyphosphate, US 4,727,102 based on melamine cyanurate, an hydroxyalkyl derivative of isocyanuric acid and ammonium polyphosphate, and published patent application WO 85/05626 based on various compounds of phosphorus and nitrogen, particularly a combination of melamine phosphate, pentaerythritol and ammonium polyphosphate.

More recent formulations comprise an organic or inorganic phosphorus compound and an organic compound containing nitrogen, generally an aminoplast resin obtained by condensation of urea, melamine, or dicyandiamide with formaldehyde.

Examples of formulations comprising two additives are reported in US patent 4,504,610 based on oligomeric derivatives of 1,3,5-triazine and ammonium polyphosphate, and European patent 14,463 based on ammonium polyphosphate and an organic compound selected from the group consisting of benzylguanamine and products of the reaction between aldehydes and various nitrogenated cyclic compounds, particularly benzylguanamine-formaldehyde copolymers.

It is also possible to have flame-retardant compositions with single-component additives containing both nitrogen and phosphorus in an organic molecule, as described in US patent 4,201,705.

These intumescent flame retardant systems confer on the polymer that contains them the property of forming a carbon residue on the application thereto of a flame. Retardant systems of this type present a number of advantages: no corrosion in the machinery used to handle the polymers, lower fume emission with respect to the systems containing metallic compounds and halogenated hydrocarbons, and above all the possibility of conferring on the polymers satisfactory flame-retardant properties with a smaller quantity of total additive, and therefore, without excessive deterioration of the physical properties of the polymers.

It has now been found that it is possible to confer high flame-retardant properties on polymers with a new class of nitrogen compounds obtained by salifying derivatives of 2,4,6-triamino-1,3,5-triazine containing two or more triazine rings with cyanuric acid.

As previously stated, 2,4,6-triamino-1,3,5-triazine cyanurate (melamine) as a coadditive in intumescent formulations of various polymers, especially polyolefins, is known in the art (US 4,727,102). However, to have flame-retardant action, the formulations must contain not only a phosphorated additive, but also a component containing the carbon necessary for the formation of the insulating carbon layer (char), such as for example, the hydroxyalkyl derivative of isocyanuric acid.

On the other hand, it has now been found that the cyanuric acid salts (cyanurates) of particular derivaties of 2,4,6-triamino-1,3,5-triazine containing two or more triazine rings, allow to obtain, just with the help of the phosphorated component, intumescent polymer compositions characterized by good thermal stability with respect ot thermal oxidation as well as during the conversion of the polymer composition into useful articles, thus allowing such conversion to be carried out at higher temperatures compared to those in the known art.

As reported in published European patent application 415,371, the derivaties of 2,4,6-triamino-1,3,5-triazine containing two or more triazine rings, whose cyanurates are comprised in the flame-retardant polymer compositions of the present invention, confer, together with ammonium or amine phosphates and/or phosphonates, excellent flame-retardant properties to the polymers that contain them. However, these additives have poor thermal stability. As can be shown by thermogravimetric analysis (T.G.A.), they lose weight when heated.

However, the polymer composition with the cyanurates of this invention has high flame-retardant levels with improved thermal stability, because said cyanurates show an excellent heat stability. Therefore, they retain a high flame-retardant property even after the polymer composition comprising them is subjected to hot processing operations.

Moreover, the cyanurates of this invention, compared to the triazine derivatives used to prepare them, are completely insoluble in water. Furthermore, in case of fire, the polymer composition of the present invention has the advantage of producing a very moderate and nondarkening fume emission.

Published European patent application 545,496 describes flame-retardant compositions comprising cyanurates of derivatives of 2,4,6-triamino-1,3,5-triazine containing only one triazine ring. However, the composition of the present invention has a higher oxygen index, at equal content of flame-retardant additives, with respect to the compositions described in this European patent application.

The present invention provides a flame-retardant polymer composition which comprises:
a) 90 to 40 parts by weight of a thermoplastic or elastomeric polymer;
b) 6 to 33 parts by weight, preferably 8 to 30 parts by weight, of at least one phosphorus compound selected from the group consisting of ammonium and amine phosphates or phosphonates; and
c) 4 to 27 parts by weight, preferably 5 to 20 parts by weight, of at least one cyanuric acid salt of a derivative of 2,4,6-triamino-1,3,5-triazine containing two or more triazine rings, said salt having the general formula(I): 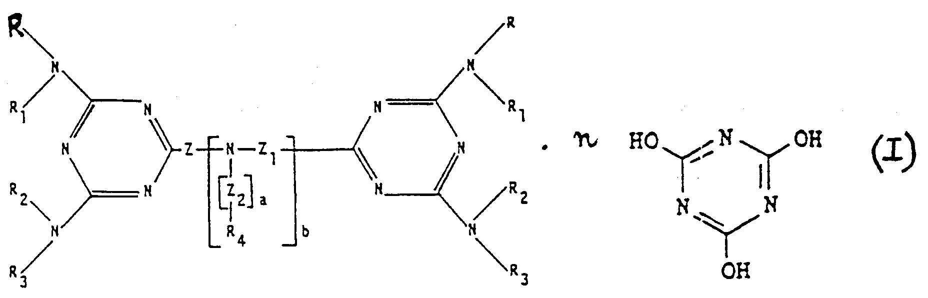 wherein:
   n is an integer from 1 to 7, preferably 1 to 3;
   the radicals from R to R₃ are the same or different, and also can be different from one triazine ring to another triazine ring, and are:
   H; C₁-C₁₈ alkyl, C₂-C₈ alkenyl; C₆-C₁₆ cycloalkyl or alkylcycloalkyl, optionally substituted with a C₁-C₄ hydroxyl or hydroxyalkyl function; 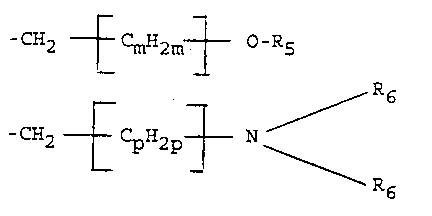 wherein:
   m = an integer from 1 to 7, preferably from 1 to 3;
   p = an integer from 1 to 5,
   R₅ = H; C₁-C₈ alkyl, preferably H or C₁-C₄ alkyl, particularly when m is from 1 to 3; C₂-C₆ alkenyl; ⁅C_{q}H_{2q}⁆O-R₇ wherein q is an integer from 1 to 4, and R₇ is H or C₁-C₄ alkyl, C₆-C₁₂ cycloalkyl or C₆-C₁₂ alkylcycloalkyl;
   the R₆ radicals can be the same or different and are:
   H; C₁-C₈ alkyl; C₂-C₆ alkenyl; C₆-C₁₂ cycloalkyl or alkylcycloalkyl; C₁-C₄ hydroxyalkyl; or the group: 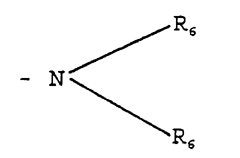 is substituted with a heterocyclic radical bonded to the alkyl chain -CH₂ [ CₚH₂ₚ ] by the nitrogen atom, and optionally containing another heteroatom preferably selected from O, S and N;
   or in the general formula (I) at least one of the groups: 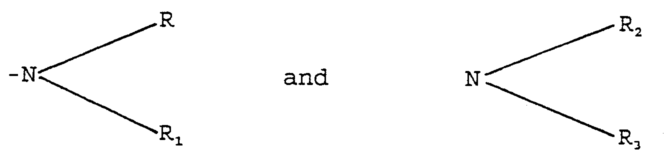 is substituted with a heterocyclic radical bonded to the triazine ring by the nitrogen atom, and optionally containing another heteroatom selected preferably from O, S and N;
   a is O or 1;
   b is O or an integer from 1 to 5;
   R₄ is H or: 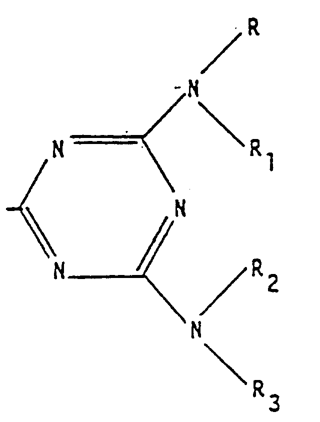 and its meaning can vary inside each repetitive unit;
   when b is O, Z is a bivalent radical of one of the following formulas II-XI: 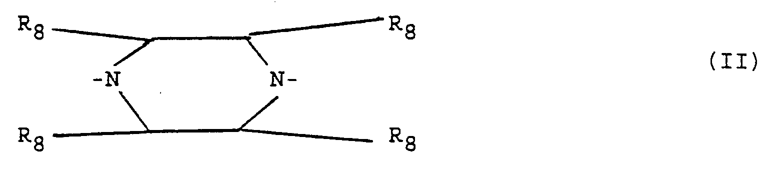 wherein the R₈ radicals, which can be the same or different, are H or C₁-C₄ alkyl; 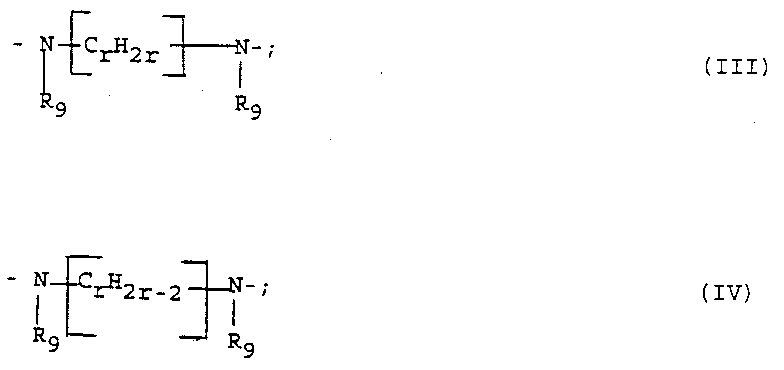 wherein r is an integer from 2 to 14; R₉ is H, C₁-C₄ alkyl, C₂-c₆ alkenyl, C₁-C₄ hydroxyalkyl; 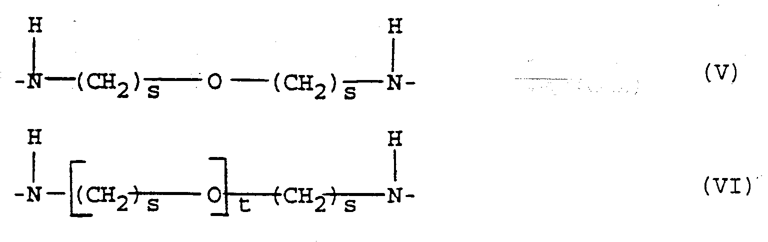 wherein s is an integer from 2 to 5, and t is an integer from 1 to 3; 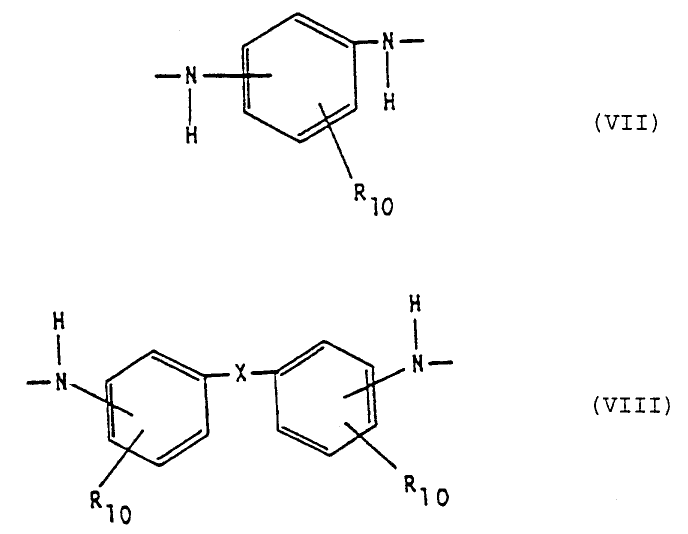 wherein:
   X is a C-C direct bond, O, S, S-S, SO, SO₂, NH, NHSO₂, NHCO, N=N, or CH₂;
   R₁₀ is H, OH, C₁-C₄ alkyl or C₁-C₄ alkoxyl; 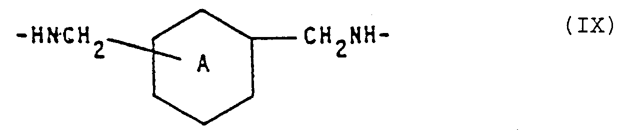 wherein A is a saturated or unsaturated cyclic ring. 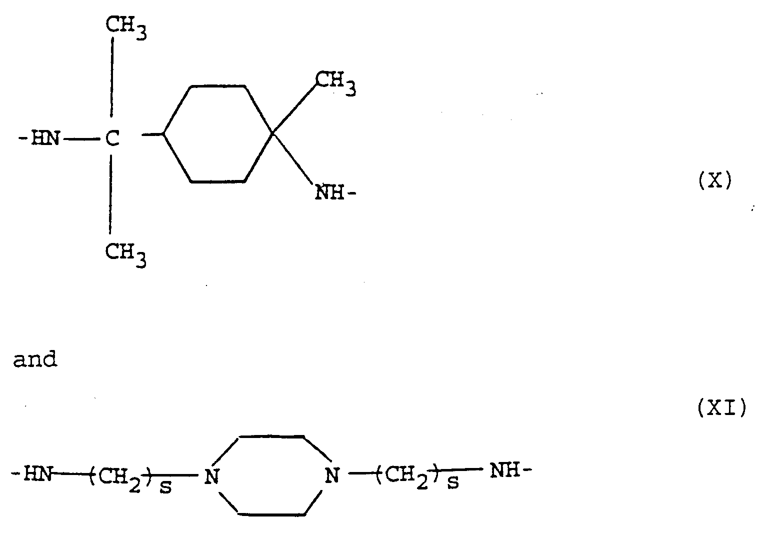 wherein s is as defined above; and when b is an integer from 1 to 5, the group: 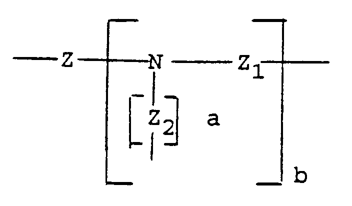 has one of the following formulas XII or XIII: 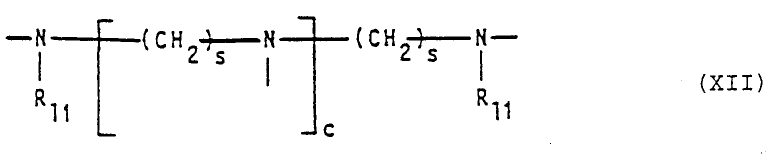 wherein:
   R₁₁ is H or C₁-C₄ alkyl;
   c is an integer from 1 to 5; and
   the s can be the same or different and are as defined above; 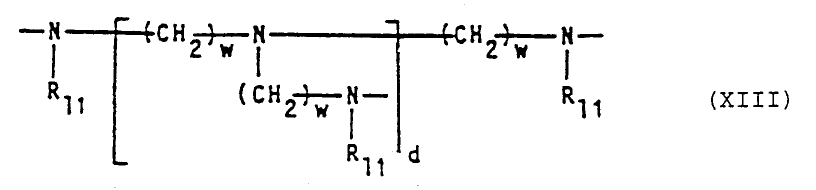 wherein:
   R₁₁ is as defined above;
   w is an integer from 2 to 4; and
   d is 1 or 2.

Under the general formula (I) are also those compounds that have an asymmetric structure, in the sense that R, R₁, R₂ and R₃ can have different meanings on each triazine ring.

Moreover, the present invention comprises salts having the general formula (I), wherein when the radicals from R to R₃ are H, Z is a bivalent radical of formula (II) or (XI), or a polyvalent radical of formula (XIII).

Examples of radicals from R to R₃ in general formula (I) are:
methyl; ethyl; propyl; isopropyl; n-butyl; isobutyl; tert-butyl; n-pentyl; isopentyl; n-hexyl; tert-hexyl, octyl, tert-octyl; decyl; dodecyl; octadecyl; ethenyl; propenyl; butenyl; isobutenyl; hexenyl; octenyl; cyclohexyl; propylcyclohexyl; butylcyclohexyl; decylcyclohexyl; hydroxycyclohexyl; hydroxyethylcyclohexyl; 2-hydroxyethyl; 2-hydroxypropyl; 3-hydroxypropyl; 3-hydroxybutyl; 4-hydroxybutyl; 3-hydroxypentyl; 5-hydroxypentyl; 6-hydroxyhexyl; 3-hydroxy-2,5-dimethylhexyl; 7-hydroxyheptyl; 7-hydroxyoctyl; 2-methoxyethyl; 2-methoxypropyl; 3-methoxypropyl; 4-methoxybutyl; 6-methoxyhexyl; 7-methoxyheptyl; 7-methoxyoctyl; 2-ethoxyethyl; 3-ethoxypropyl; 4-ethoxybutyl; 3-propoxypropyl; 3-butoxypropyl; 4-butoxybutyl; 4-isobutoxybutyl; 5-propoxypentyl; 2-cyclohexyloxyethyl; 2-ethenyloxyethyl; 2-(N,N-dimethylamino)ethyl; 3-(N,N-dimethylamino)propyl; 4-(N,N-dimethylamino)butyl; 5-(N,N-dimethylamino)pentyl; 4-(N,N-diethylamino)butyl; 5-dimethylamino)pentyl; 5-(N,N-diisopropylamino)pentyl; 3-(N-ethylamino)propyl; 4-(N-methylamino)butyl; 4-(N,N-dipropylamino)butyl; 2-(N,N-diisopropylamino)ethyl; 6-(N-hexenylamino)hexyl; 2-(N-ethenylamino)ethyl; 2-(N-cyclohexylamino)ethyl; 2-(N-2-hydroxyethylamino)ethyl; 2-(2-hydroxyethoxy)ethyl; 2-(2-methoxyethoxy)ethyl; 6-(N-propylamino)hexyl.

Examples of heterocyclic radicals that can substitute the groups: 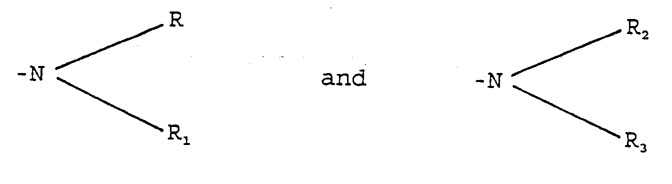
are:
aziridinyl; pyrrolidinyl; piperidinyl; morpholinyl; thiomorpholinyl; piperazinyl; 4-methylpiperazinyl; 4-ethylpiperazinyl; 2-methylpiperazinyl; 2,5-dimethylpiperazinyl; 2,3,5,6-tetramethylpiperazinyl; 2,2,5,5-tetramethylpiperazinyl; 2-ethylpiperazinyl; 2,5-diethylpiperazinyl.

Examples of heterocyclic radicals that can substitute the group: 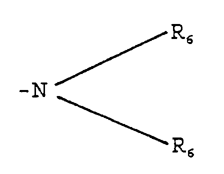
are:
aziridinyl; pyrrolidinyl; piperidinyl; morpholinyl; thiomorpholinyl; piperazinyl; 4-methylpiperazinyl; 4-ethylpiperazinyl.

Examples of bivalent radical represented by -Z- are those derived by elimination of one of the hydrogen atoms from each amino group of the following diamine compounds: piperazine; 2-methylpiperazine; 2,5-dimethylpiperazine; 2,3,5,6-tetramethylpiperazine; 2-ethylpiperazine; 2,5-diethylpiperazine; 1,2-diaminoethane; 1,3-diaminopropane; 1,4-diaminobutane; 1,5-diaminopentane; 1,6-diaminohexane; 1,8-diaminooctane; 1,10-diaminodecane; 1,12-diaminododecane; N,N'-dimethyl-1,2-diaminoethane; N-methyl-1,3-diaminopropane; N-ethyl-1,2-diaminoethane; N-isopropyl-1,2-diaminoethane; N-(2-hydroxyethyl)-1,2-diaminoethane; N,N'-bis(2-hydroxyethyl)-1,2-diaminoethane; N-(2-hydroxyethyl)-1,3-diaminopropane; N-hexenyl-1,6-diaminohexane; N,N'-diethyl-1,4-diamino-2-butene; 2,5-diamino-3-hexene; 2-aminoethylether; (2-aminoethoxy)methylether; 1,2-bis-(2-aminoethoxy)ethane; 1,3-diaminobenzene; 1,4-diaminobenzene; 2,4-diaminotoluene; 2,4-diaminoanisole; 2,4-diaminophenol; 4-aminophenylether; 4,4'-methylenedianiline; 4,4'-diaminobenzanilide; 3,3'-diaminodiphenylsulfone (also named as 3-aminophenylsulfone); 4,4'-diaminodiphenylsulfone (also named as 4-aminophenylsulfone); 4,4'-diaminophenylsulfoxide (also named as 4-aminophenylsulfoxide); 4-aminophenyldisulfide; 1,3-bis(aminomethyl)benzene; 1,4-bis(aminomethyl)benzene; 1,3-bis(aminomethyl)cyclohexane; 1,8-diamino-p-methane; 1,4-bis(2-aminoethyl)piperazine; 1,4-bis(3-aminopropyl)piperazine; 1,4-bis(4-aminobutyl)piperazine; 1,4-bis(5-aminopentyl)piperazine.

Examples of polyvalent radicals: 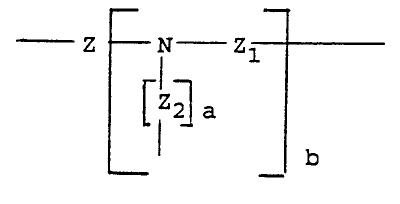
are those derived, by elimination of a hydrogen atom from each reacted aminic group of the following polyamine compounds:
bis(2-aminoethyl)amine; bis(3-aminopropyl)amine; bis(4-aminobutyl)amine; bis(5-aminopentyl)amine; bis[2-(N-methylamino)ethyl]amine; 2-N-butyl-bis(2-aminoethyl)amine; bis[3-(N-methylamino)propyl]amine; N-(3-aminopropyl)-1,4-diaminobutane; N-(3-aminopropyl)-1,5-diaminopentane; N-(4-aminobutyl)-1,5-diaminopentane; tris(2-aminoethyl)amine; tris(3-aminopropyl)amine; tris(4-aminobutyl)amine; tris[2-(N-ethylamino)ethyl]amine; N,N'-bis(2-aminoethyl)-1,2-diaminoethane; N,N'-bis(3-aminopropyl)-1,3-diaminopropane; N,N'-bis(2-aminoethyl)-1,3-diaminopropane; N,N'-bis(3-aminopropyl)-1,2-diaminoethane, N,N'-bis-(3-aminopropyl)-1,4-diaminobutane; bis [2-(2-aminoethyl)aminoethyl]amine; N,N'-bis[2-(2-aminoethyl)aminoethyl]-1,2-diaminoethane; N,N'-bis[3-(2-aminoethyl)aminopropyl]-1,2-diaminoethane;N,N,N',N'-tetrakis(2-aminoethyl)-1,2-diaminoethane.

Specific compounds within formula (I) are disclosed in the examples set forth herein.

The cyanurates of general formula (I) can be synthesized by causing the reaction, preferably in the presence of a suitable solvent (such as water, methyl alcohol, ethyl alcohol and acetonitrile, for example) at a temperature from 0°C to the boiling point of the solvent used, of one mole of the corresponding derivative of 2,4,6-triamino-1,3,5-triazine having the general formula (XIV): 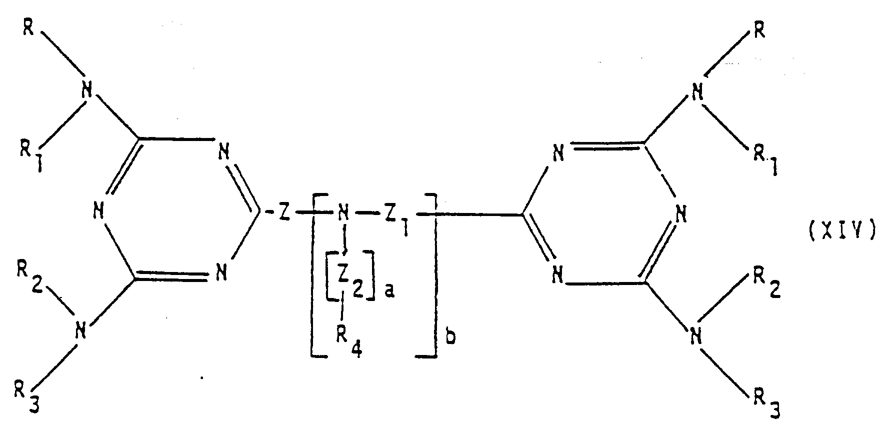
wherein the substituents R, R₁, R₂, R₃, R₄, and the radical: 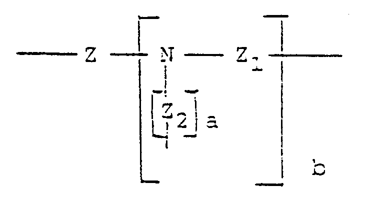
are as defined above, with n moles of cyanuric acid, where n is as previously defined in formula (I).

The cyanurate product thus formed can be easily separated from the reaction mixture by filtration, or by distilling off the solvent.

Generally cyanurates of general formula (I) are obtained as white crystalline powders, which can be used in flame-retardant polymer compositions without further purification.

Derivatives of general formula (XIV) are known; they can be synthesized readily according to the methods described in published European patent application 415,371.

Particularly preferred are the compounds of general formula (I) wherein at least one of the groups: 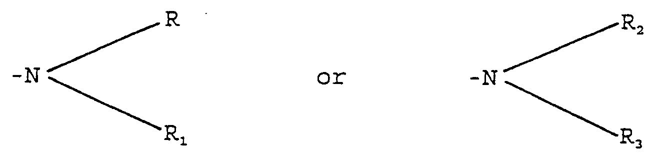
is an NH₂ radical.

The preferred phosphates are the ammonium polyphosphates of the general formula (NH₄)ₙ₊₂ Pₙ O₃ₙ₊₁ wherein n in this formula is an integer equal to or greater than 2; preferably the molecular weights of the polyphosphates must be sufficiently high to guarantee a low solubility in water. As a way of example, n preferably ranges from 2 to 500.

The composition of the polyphosphates having the formula indicated above, wherein n is a number sufficiently high and preferably ranging from 50 to 500, is basically the one that corresponds to metaphosphates (NH₄PO₃)ₙ.

An example of said polyphosphates is the one marketed as "Exolit 422" by Hoechst, and having the composition (NH₄PO₃)ₙ, where n is greater than 50. Another example is the product marketed as "Phos-Chek P/40" by Monsanto Chemical and having a similar composition.

Another polyphosphate that gives satisfactory results, especially because of its low solubility in water, is the one marketed as "Exolit 462", by Hoechst, which comprises the Exolit 422 polyphosphate composition microencapsulated in melamine-formaldehyde resin.

Other usable phosphates are those derived from amines, such as for example dimethylammonium and diethylammonium phosphates, ethylenediamine phosphate, and melamine ortho- or pyrophosphates.

Optimum results are obtained with ammonium phosphonates (mono or poly substituted) derived from mono- and polyphosphonic acids, such as for example: ethane-1,1,2-triphosphonic acid; 2-hydroxyethane-1,1,2-triphosphonic acid; propane-1,2,3-triphosphonic acid; methylphosphonic acid; ethylphosphonic acid; n-propylphosphonic acid; n-butylphosphonic acid; phenylphosphonic acid; 1-aminoethane-1,1-diphosphonic acid; 1-hydroxyethane-1,1-diphosphonic acid; 1-hydroxydodecane-1,1-diphosphonic acid; phosphonoacetic acid; 2-phosphonopropionic acid; 3-phosphonopropionic acid; 2-phosphonobutyric acid; 4-phosphonobutyric acid; aminotris(methylenephosphonic) acid; ethylenediaminotetra(methylenephosphonic) acid; hexamethylenediaminotetra(methylenephosphonic) acid; diethylenetriaminopenta(methylenephosphonic) acid.

The preferred polymers that can be used in the polymer compositions of the present invention include the polymers or copolymers of olefins of the general formula R-CH=CH₂, wherein R is a H, C₁-C₈ alkyl, or C₆-C₈ aryl radical, preferably phenyl, particularly:
1. polypropylene with isotactic index greater than 80;
2. high density polyethylene (HDPE), linear low density polyethylene (LLDPE), low density polyethylene (LDPE);
3. crystalline propylene copolymers with minor proportions (in particular up to 20% by weight) of ethylene, other C₄-C₈ α-olefins, such as for example 1-butene, 1-hexene, 1-octene and 4-methyl-1-pentene, or mixtures of ethylene and the C₄-C₈ α-olefins;
4. heterophasic olefin polymer compositions comprising (A) a crystalline homopolymer fraction of propylene or a copolymer of item (3), and (B) a copolymer fraction comprising an elastomeric copolymer of ethylene with a C₃-C₈ α-olefin, and optionally a minor proportion of a diene, where the C₃-C₈ α-olefin is preferably selected from propylene and 1-butene;
5. elastomeric copolymers of ethylene, C₃-C₈ α-olefins, preferably selected from propylene and 1-butene, and optionally a minor proportion of diene.

Examples of dienes most commonly used in the above mentioned elastomeric copolymers are butadiene, ethylidene-norbornene and 1,4-hexadiene. Said dienes, when present, are typically in an amount of from 0.5 to 10% by weight with respect to the weight of the elastomeric copolymer.

Of the olefin polymers of formula R-CH=CH₂ wherein R is aryl, the preferred ones are "crystal" and impact resistant polystyrene.

Other examples of polymers commonly used are acrylonitrile/butadiene/styrene (ABS) and styrene/acrylonitrile (SAN) copolymers; polyurethane (polyester and polyether); polyethylene terephthalate; polybutylene terephthalate; and polyamides.

The flame-retardant polymer compositions of this invention can be prepared according to known methods. For example, in one method the ammonium or amine phosphate and/or phosphonate is intimately mixed with one or more compounds of general formula (I) and finally ground, preferably to particles having a diameter smaller than 70 micrometers. This mixture is added to the polymer in a turbo-mixer to form a homogeneous blend which is then extruded and pelletized. The product thus obtained can be converted in various articles by any known molding technique.

The flame-retardant additives of the present invention are suitable for use in flame retardant paints.

Compounds comprised of general formula (I) that are not illustrated by the examples, but are equally suitable for use in the flame-retardant polymer compositions of the present invention, are those reported in Table 1, wherein R₄, when present, is a triazine ring compound of formula:

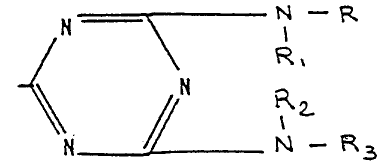

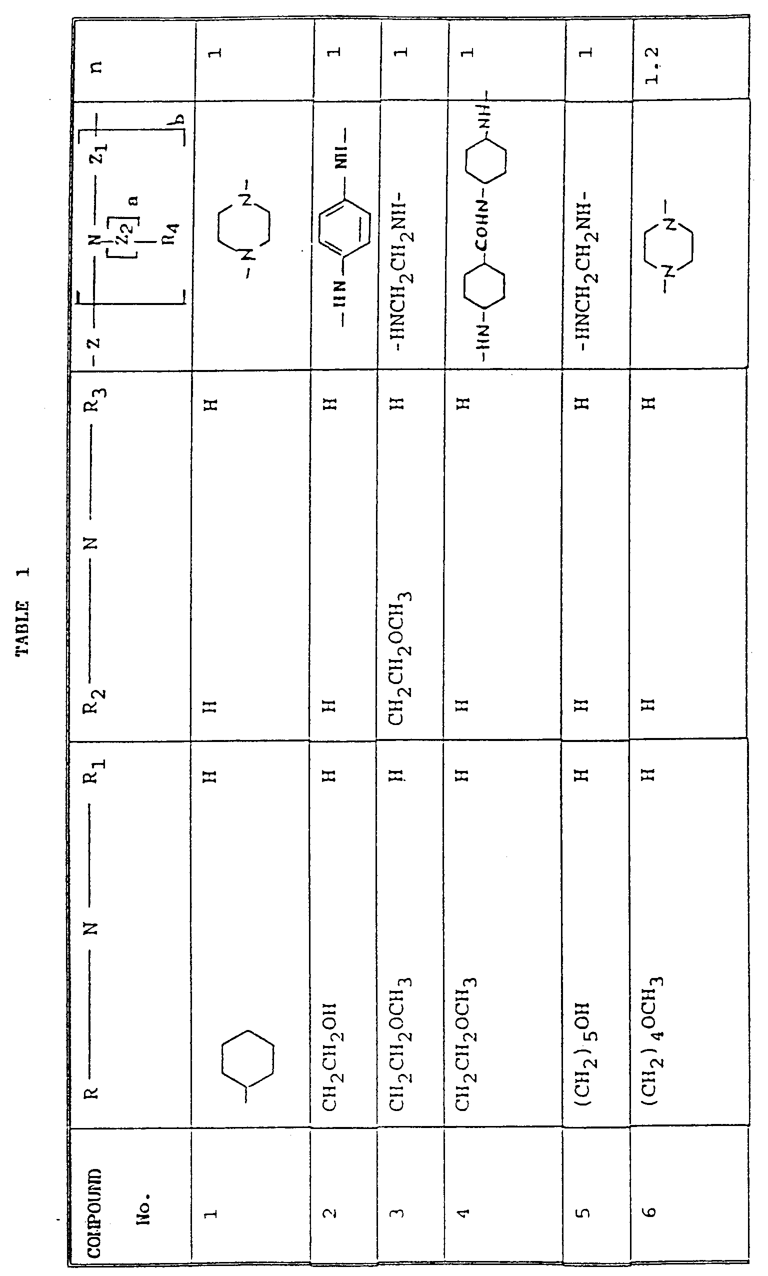

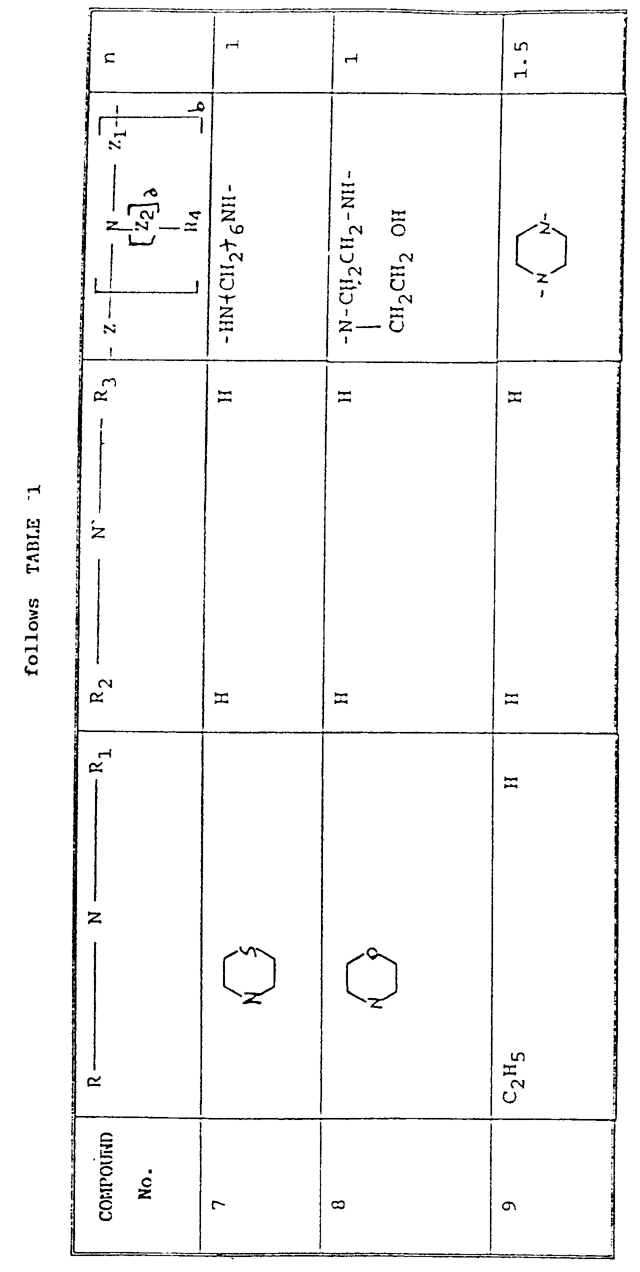

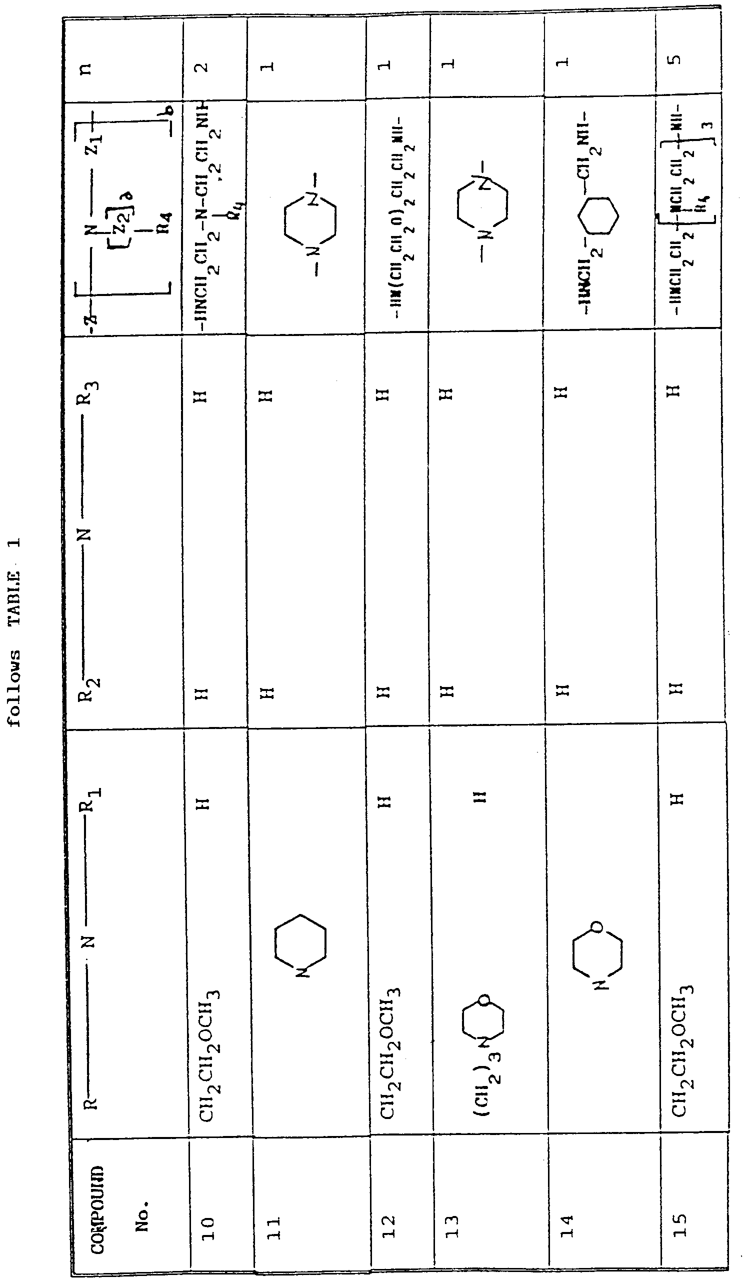

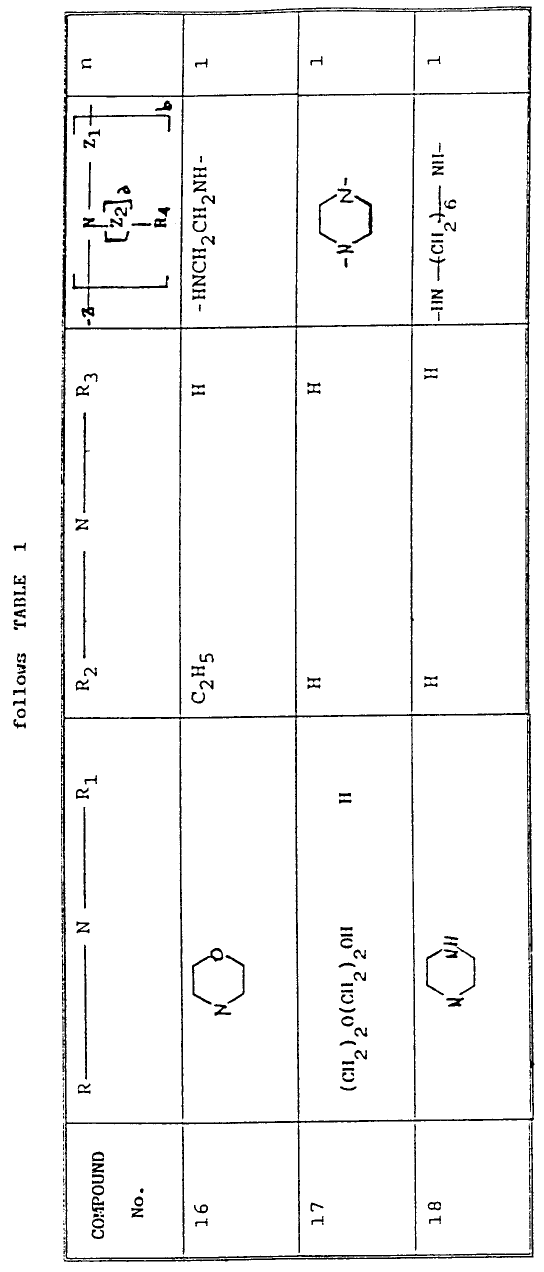

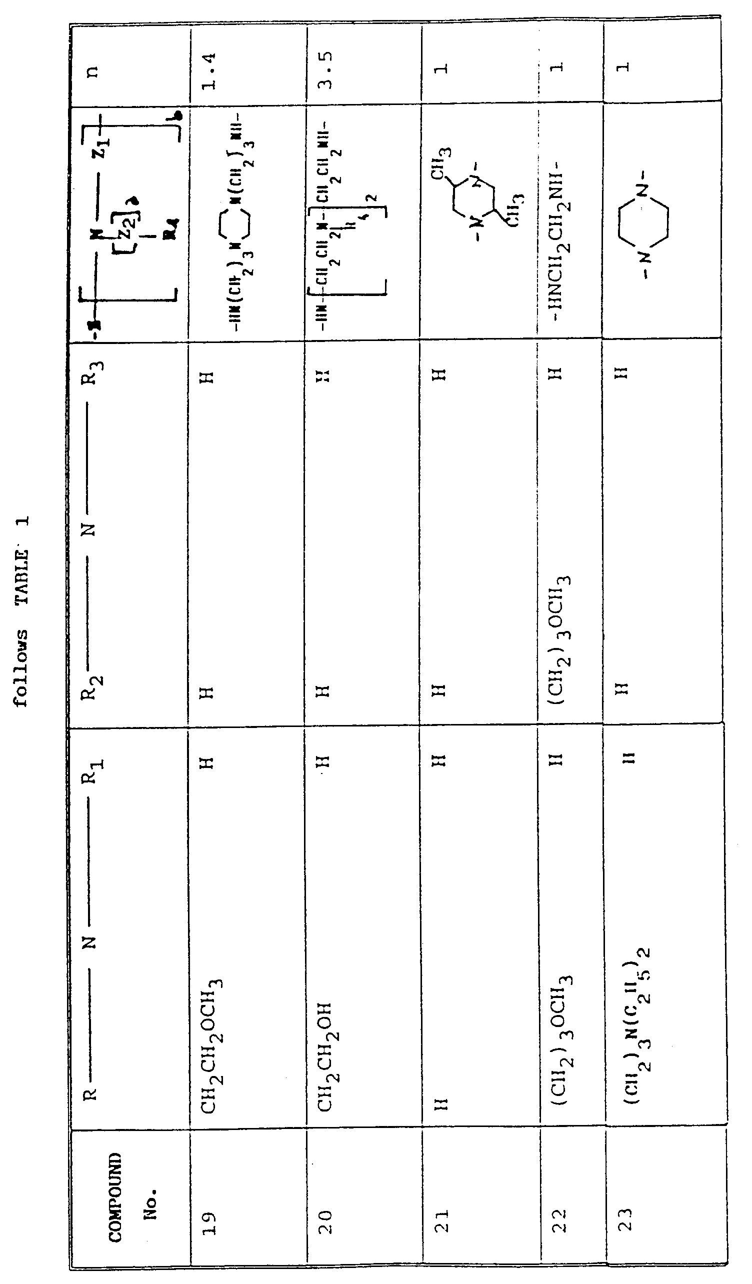

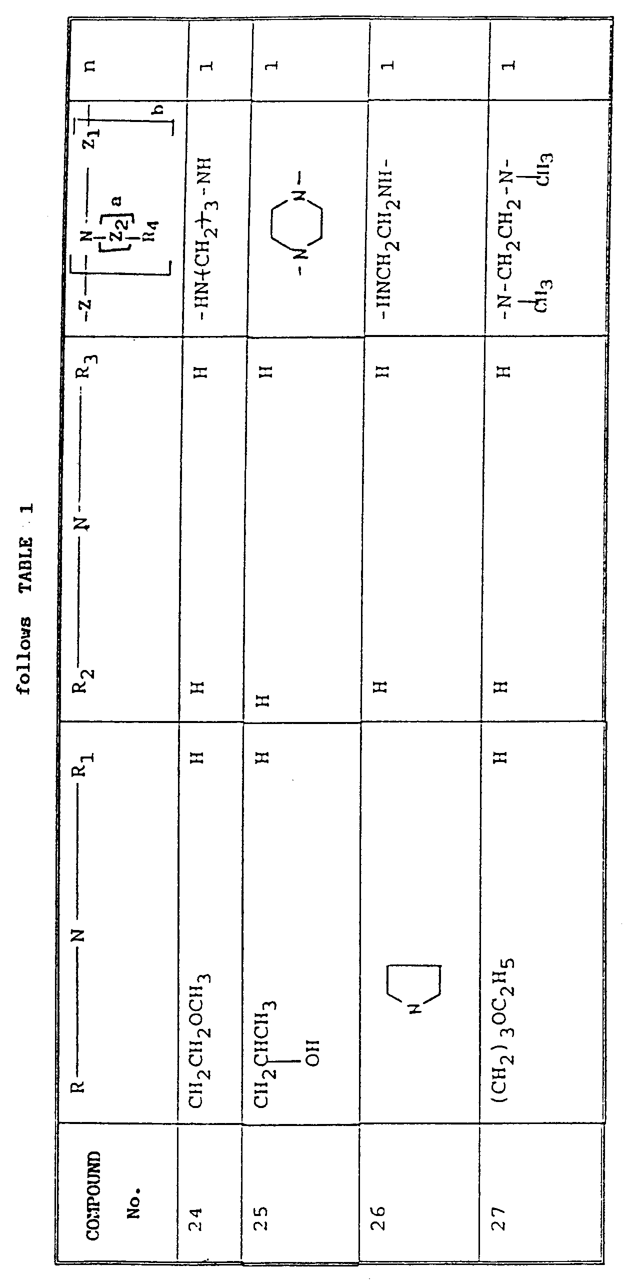

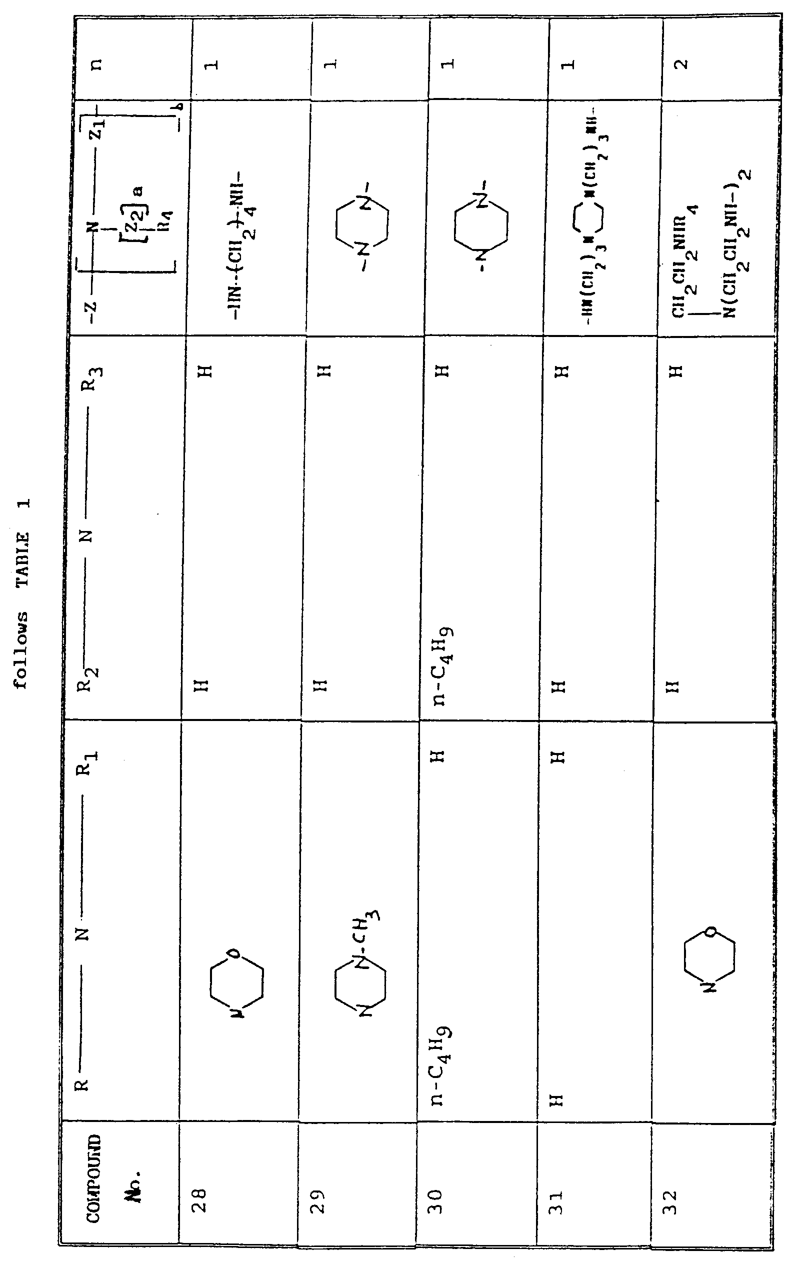

The examples below illustrate specific embodiments of the invention without limiting the same.

The salification reactions between the derivatives of general formula (XIV) and cyanuric acid are confirmed by IR spectroscopic analysis carried out on an IR Perkin Elmer 580 B grating spectrophotometer. In fact, it has been observed that an optimum reference sign is the band relative to the deformation outside the plane of the triazine ring: said band is located at about 830-800 cm⁻¹ for the undisturbed ring, and at 796-760 cm⁻¹ for the ring salified on the amino groups.

### EXAMPLE 1

Into a 3 liter reactor, complete with agitator, thermometer, feed funnel, reflux condenser and cooling bath, are introduced 184.5 g of cyanuric acid chloride and 1300 ml of methylene chloride. While externally cooling are introduced simultaneously, into the reactor over a period of three hours while maintaining the pH of the reactor between 5 and 7 and the temperature between 0 and 3°C, 75 g of 2-methoxyethylamine and 40 g of sodium hydrate dissolved in 150 ml of water. The resulting reaction mixture is kept at a temperature from 0 to 3°C for an additional three hours, and then the aqueous phase is separated therefrom. The organic solution is treated with two portions of water of 200 ml each, both times separating the aqueous phase.

After removing the methylene chloride by distillation, 217.5 g of the intermediate (XV): 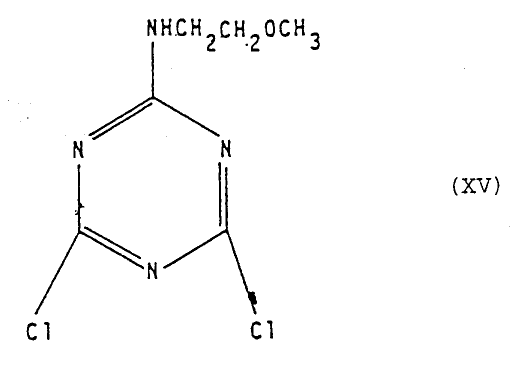
are obtained in the form of a white crystalline powder having a melting point (m.p.) = 73-75°C and a chlorine content equal to 31.68% (theoretical: 31.84%).

Into a 1 liter reactor, equipped with agitator, thermometer, feed funnel, reflux codenser and heating bath, are introduced 400 ml of acetone and 133.8 g of intermediate (XV).
The content is stirred while heating to 40°C until a solution is obtained, then 102 g of ammonia solution at 30% by weight are added in a period of 30 minutes maintaining the temperature at 40°C. The solution is then heated to 45°C and maintained at this temperature for 4 hours. After cooling to 10°C the product is filtered and washed on the filter with cold water. After drying in a 100°C oven, 114 g of intermediate (XVI) are obtained: 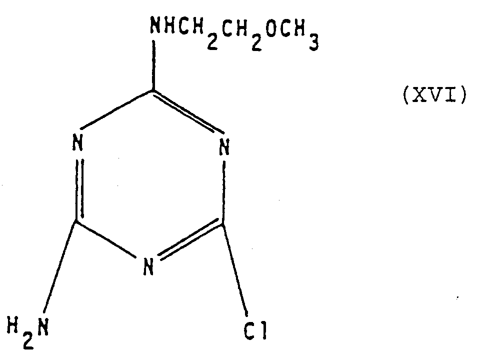
as a white crystalline powder having a m.p. of 195-197°C and a chlorine content equal to 17.18% (theoretical: 17.44%).

Into the same 1 liter reactor are introduced 500 ml of xylene, 81.4 g of the intermediate (XVI) and 17.2 g of piperazine. The resulting mixture is heated to 100°C and maintained at this temperature for 2 hours. 16 g of sodium hydrate are added and the reactor contents are heated to the boiling point. The contents are then refluxed for 20 hours, cooled to ambient temperature and filtered. The filter cake is washed several times with water and then dried. 74.2 g of the intermediate (XVII) are obtained: 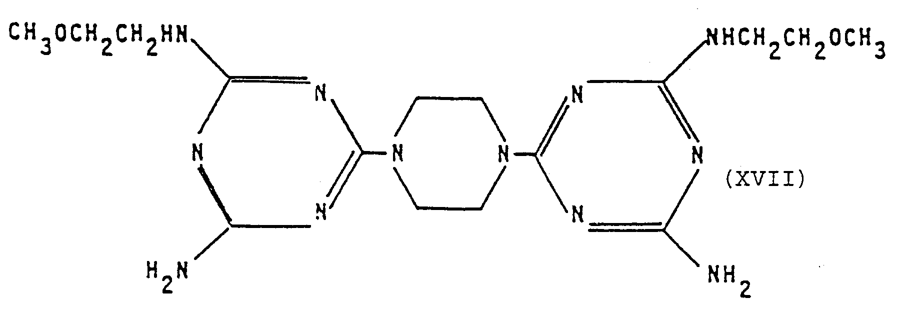
as a white crystalline powder having a m.p. of 212-215°C.

The structure of intermediates (XV), (XVI) and (XVII) was confirmed by IR spectroscopic analysis.

Into the same 1 liter reactor are introduced 500 ml of water and, while stirring, 84.0 g of intermediate (XVII) and 25.8 g of cyanuric acid. The reactor contents are heated to the boiling point, refluxed for 16 hours, and cooled to ambient temperature. The product thus formed is filtered, and the filter cake washed on the filter with water.

By drying the filter cake in a 100°C oven, 108.4 g of product: 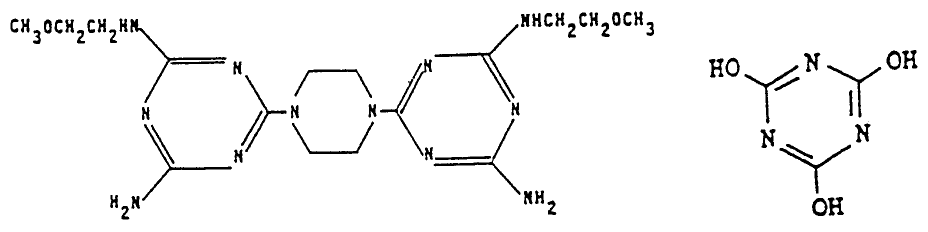
are obtained as a white crystalline powder having a m.p. of 276-280°C.

### EXAMPLE 2

Into the same 3 liter reactor of Example 1 184.5 g of the cyanuric acid chloride and 1300 ml of methylene chloride are introduced. Proceeding as described in Example 1, but using 87.2 g of morpholine, 230 g of the intermediate (XVIII) are obtained: 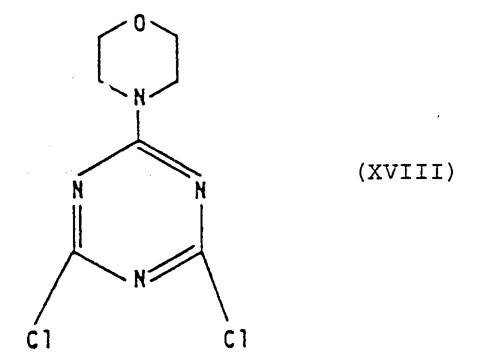
as a white crystalline powder having a m.p. of 155-157°C and a chlorine content equal to 29.87% (theoretical: 30.21%).

Into a 0.5 liter reactor, equipped as in Example 1, are introduced 100 g of an ammonia solution at 30% by weight, 100 ml of water and 70.5 g of intermediate (XVIII). The reactor contents are heated to 50°C and maintained at this temperature for 7 hours. The reactor mixture is then cooled to ambient temperature, filtered and the filter cake washed several times with water. After drying 58 g of the intermediate (XIX) are obtained: 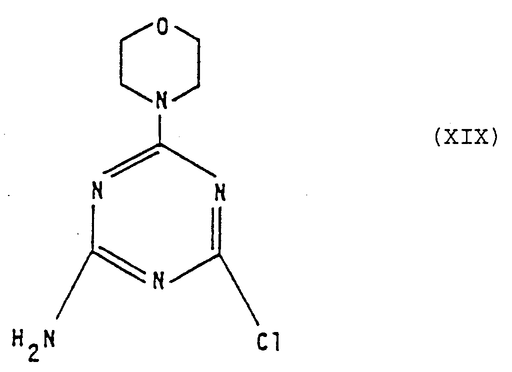
as a white crystalline powder having a m.p. of 189-191°C and a chlorine content equal to 16.28% (theoretical: 16.47%).

Into a 1 liter reactor equipped as described above, are introduced 600 ml of ortho-dichlorobenzene, 107.8 g of intermediate (XIX) and 15.0 g of ethylenediamine. The reactor contents is heated to 100°C and maintained at this temperature for 2 hours. Then 20 g of sodium hydrate are added, and the mixture is heated to 140°C for 16 hours, after which it is cooled to ambient temperature, filtered and washed repeatedly with water. After drying, one obtains 99.8 g of the intermediate (XX): 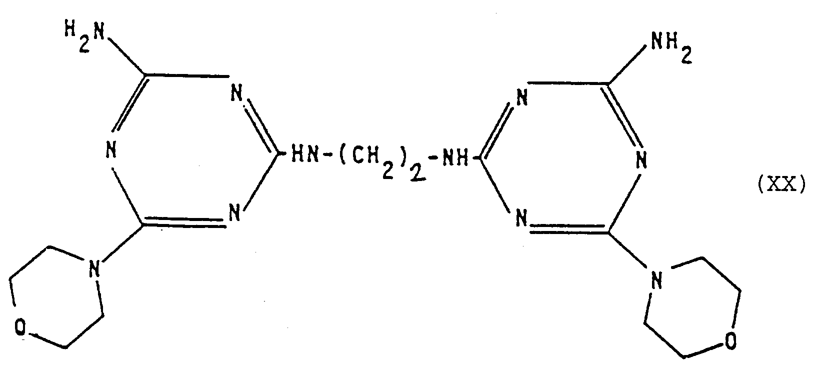
a white crystalline powder having a m.p. = 265-268°C.

The structures of intermediates (XVIII), (XIX), and (XX) were confirmed by IR spectroscopic analysis.

Into the same 1 liter reactor are introduced 450 ml of water and, while stirring, 62.7 g of intermediate (XX) and 19.4 g of cyanuric acid. The reactor contents are heated to the boiling point and maintained in reflux for 18 hours. After cooling to ambient temperature the product thus formed is filtered and the filter cake washed several times with water. After drying one obtains 81.4 g of product: 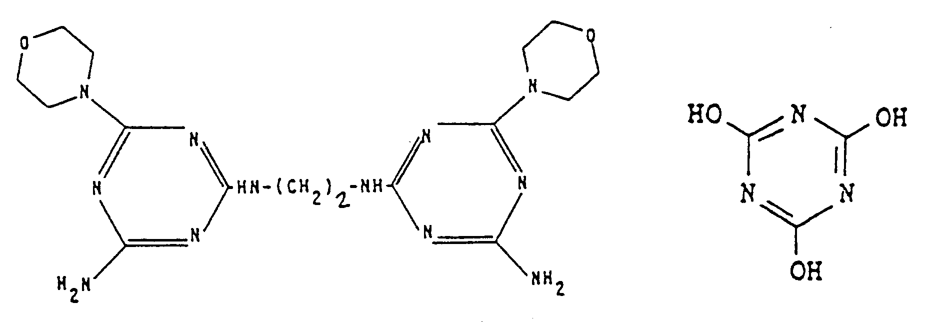
as a white crystalline powder having a m.p. higher than 300°C.

### EXAMPLE 3

Into a 3 liter reactor equipped with an agitator, thermometer, feed funnel, reflux condenser and heating bath, 184.5 g of cyanuric acid chloride and 800 ml of acetone are introduced. The reactor contents are heated to 40°C while stirring until a solution is obtained, then 284 g of an ammonia solution at 30% by weight is added over a period of 1 hour and 30 minutes, while maintaining the temperature at 40°C. Then the resulting solution is heated to 45°C, and maintained at this temperature for 4 hours. After cooling to ambient temperature, the product thus formed is filtered, and then the filter cake washed several times with water. After drying in a 50-60°C oven under vacuum, 113 g of the intermediate (XXI) are obtained 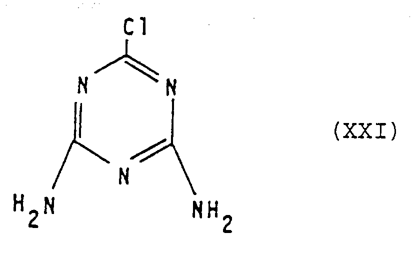
as a white crystalline powder having a chlorine content equal to 24.2% (theoretical: 24.4%).

Into a 1 liter reactor, equipped as the one above, are introduced 400 ml of xylene, 58.2 g of intermediate (XXI) and 17.2 g of piperazine. The reactor contents are heated to 100°C and maintained at this temperature for 2 hours. 16 g of solid sodium hydrate are then added, and the contents are heated to the boiling point. It is maintained in reflux for about 20 hours, and then cooled to ambient temperature and filtered. The filter cake is then washed several times with water and dried, thus obtaining 54.2 g of the intermediate (XXII): 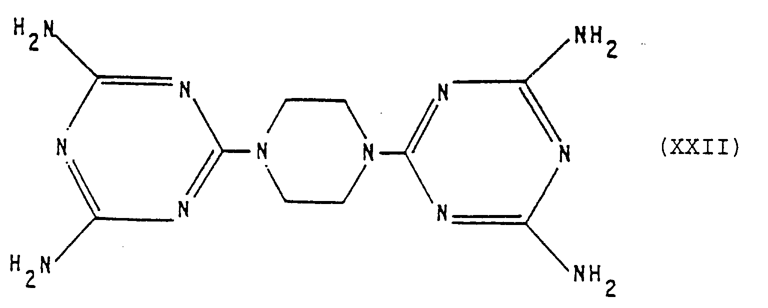
as a white crystalline powder having a m.p. greater than 300°C.

The structures of intermediates (XXI) and (XXII) were confirmed by IR spectroscopic analysis.

Into a 1 liter reactor, equipped as the one above, are introduced 450 ml of water, and while stirring, 45.6 g of intermediate (XXII) and 19.4 g of cyanuric acid. The mixture is brought to the boiling point, and maintained in reflux for 16 hours. It is then cooled to ambient temperature, then the product thus formed is filtered, and the filter cake washed several times with water.
After drying the filter cake at 100°C in a oven one obtains 63.4 g of product: 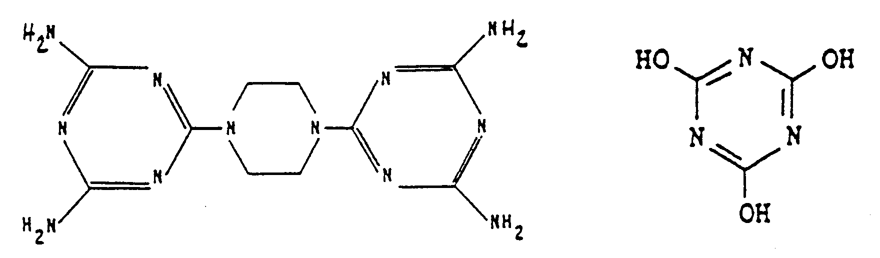
as a white crystalline powder having a m.p. greater than 300°C.

### EXAMPLE 4

In a 1 liter reactor equipped as in Example 1, are introduced 450 ml of water, 91.6 g of intermediate (XVI), and, while stirring, 21.9 g of tris(2-aminoethyl)amine. The mixture is heated to 80°C and maintained at this temperature for 3 hours. Then 18 g of sodium hydrate dissolved in 30 cm³ of water are added, and the reactor contents are heated to the boiling point. It is maintained in reflux for 16 hours, then cooled to 10°C, and the product thus formed is filtered and the filter cake washed several times with cold water. After drying the filter cake at 100°C in an oven, 85.4 g of intermediate (XXIII) are obtained 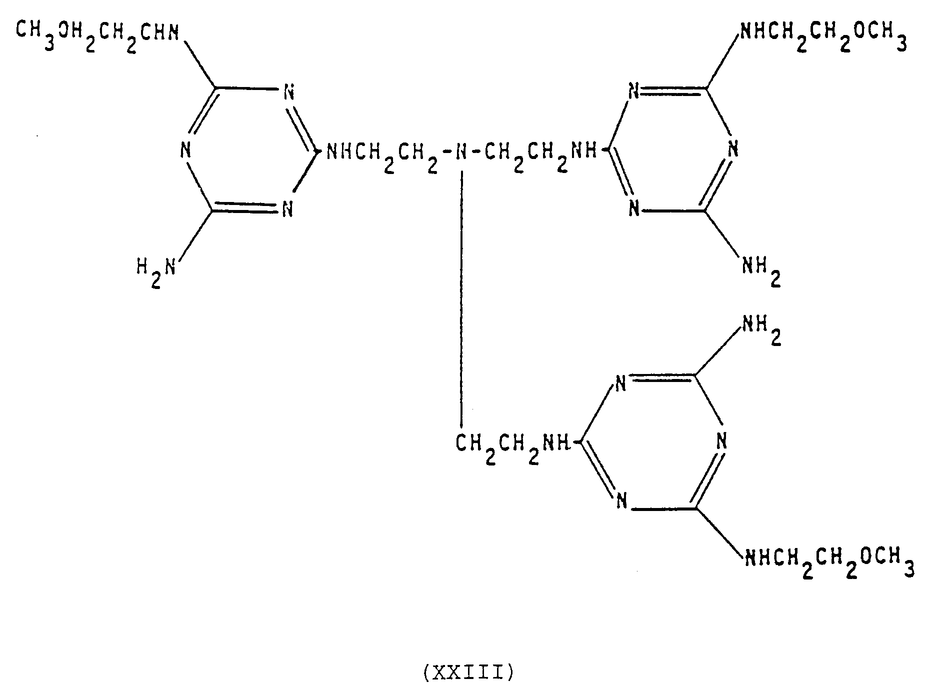
as a white crystalline powder having a m.p. of 190-195°C.

The structure of intermediate (XXIII) was confirmed by IR spectroscopic analysis.

Into the same 1 liter reactor are introduced 600 ml of water and, while stirring, 64.7 g of intermediate (XXIII) and 25.8 g of cyanuric acid are added. The mixture is heated to the boiling point and maintained in reflux for 18 hours. The product is filtered, washed and dried as in Example 1, thus obtaining 88.7 g of product: 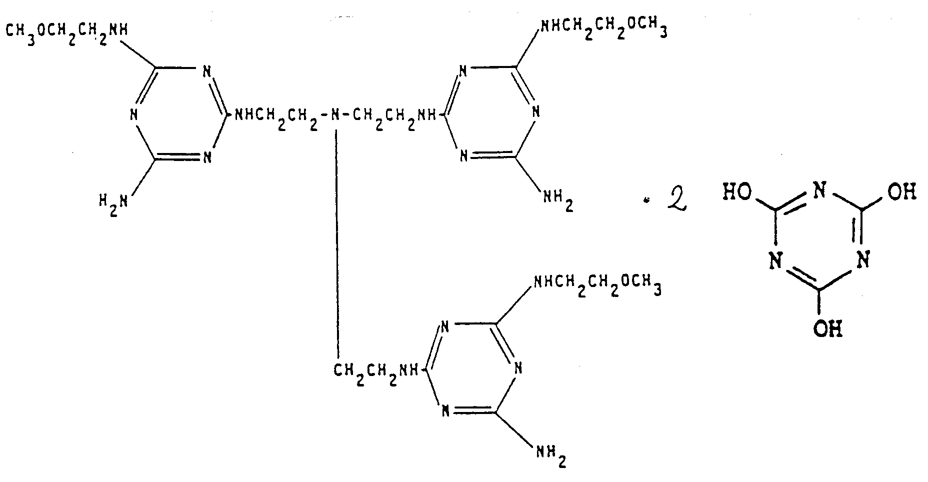
as a white crystalline powder having a m.p. greater than 300°C.

### EXAMPLE 5

Into a 1 liter reactor, equipped as in Example 1, are introduced 500 ml of xylene, 86.2 g of intermediate (XIX) and 15.1 of tetraethylenepentamine. The mixture is heated to 80°C and is maintained at this temperature for 2 hours. Then 16 g of sodium hydrate are added and the temperature is brought to 110°C. The mixture is maintained at 110°C for 18 hours, then it is cooled to ambient temperature, and the product formed is filtered and abundantly washed on the filter with water. After drying the filter cake at a 100°C in an oven, 82.6 g of intermediate (XXIV) are obtained: 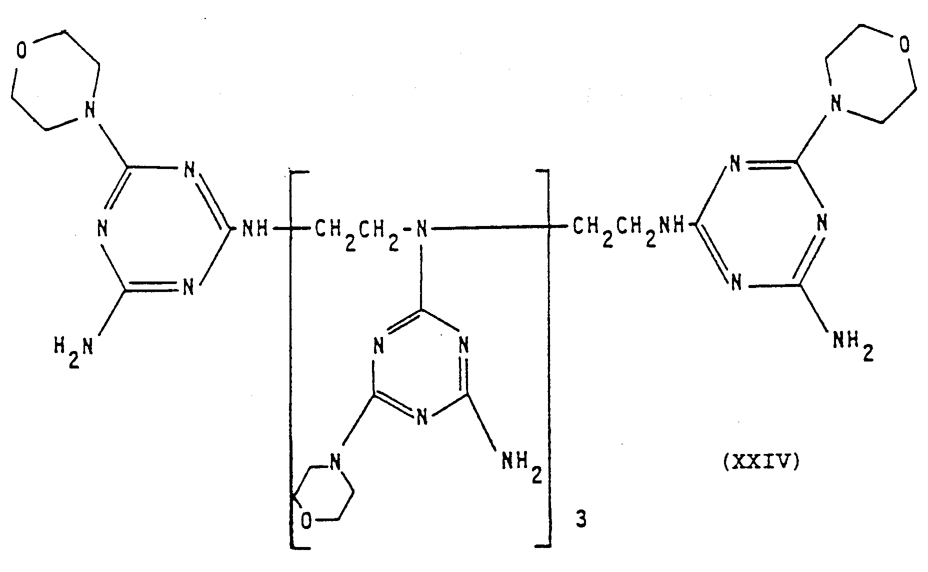
as a white crystalline powder having a m.p. of 178-183°C. The structure of intermediate (XXIV) was confirmed by IR spectroscopic analysis.

Into the same 1 liter reactor are introduced 500 ml of water, and, while stirring, 65.0 g of intermediate (XXIV) and 23.2 g of cyanuric acid. The reactor mixture is heated to the boiling point and maintained in reflux for 18 hours. It is then cooled to ambient temperature, the product thus formed is filtered, and the filter cake washed several times with water. After drying the filter cake at 100°C in an oven, 87.1 g of product are obtained: 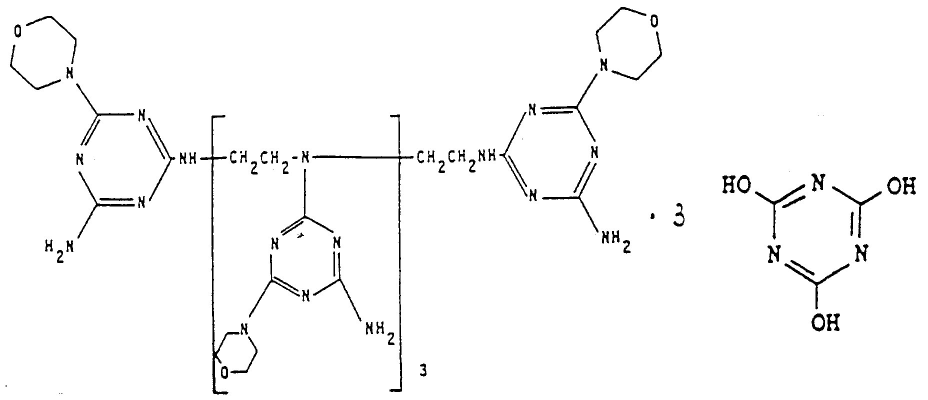
as a white crystalline powder having a m.p. greater than 300°C.

### EXAMPLE 6

Into the same 1 liter reactor of the preceding Example are introduced 400 ml of water, and, while stirring, 86.2 g of intermediate (XIX) and 20.6 g of diethylenetriamine. The mixture is heated to 80°C for 2 hours, then 16 g of sodium hydrate dissolved in 30 ml of water are added, and the reactor contents are brought to the boiling point. The contents are maintained in reflux for about 14 hours, then cooled, filtered, washed and dried as described in the Example above, thus obtaining 86.2 g of intermediate (XXV): 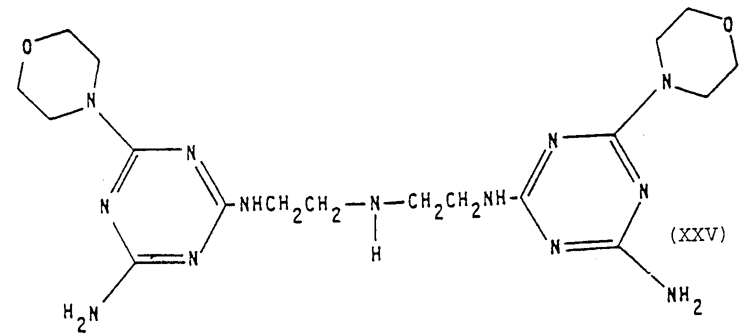
as a white crystalline powder having a m.p. of 198-201°C.

Into the same 1 liter reactor are introduced 600 of water and, while stirring, 69.1 g of intermediate (XXV) and 19.4 g of cyanuric acid. The reactor contents are heated to the boiling point and maintained in reflux for 12 hours, then cooled, filtered, washed and dried as described in the above Examples, thus obtaining 86.8 g of product: 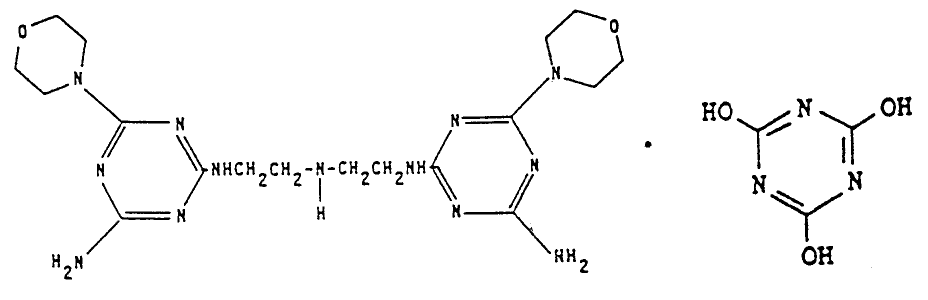
as a white crystalline powder having a m.p. of 206-209°C.

### EXAMPLE 7

Into a 1 liter reactor equipped as in Example 1, are introduced 500 of water, and, while stirring, 29.2 g of tris(2-aminoethyl)amine and 87.3 g of intermediate (XXI).

The reactor contents are heated to 50°C and maintained at this temperature for 1 hour. Then, over a period of 3 hours, 24.0 g of sodium hydrate dissolved in 50 ml of water are introduced, while at the same time increasing the temperature of the mixture to the boiling point. The reactor contents are maintained in reflux for about 10 hours, then cooled to ambient temperature and filtered. The filter cake is washed several times with water and dried. 89.4 g of intermediate (XXVI) are obtained: 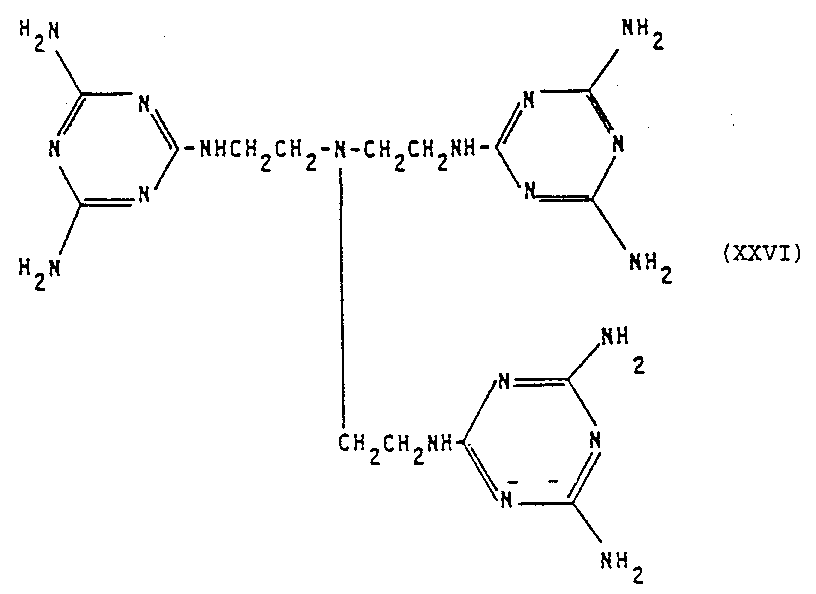
as a white crystalline powder having a m.p. of 125-130°C. The structure of intermediate (XXVI) was confirmed by IR spectroscopic analysis.

Into the same 1 liter reactor are introduced 600 ml of water and, while stirring, 47.3 g of intermediate (XXVI) and 38.7 g of cyanuric acid. The contents are heated to the boiling point and maintained in reflux for 18 hours, and then cooled, filtered, washed and dried as described in the preceding Example, thus obtaining 85.2 g of product: 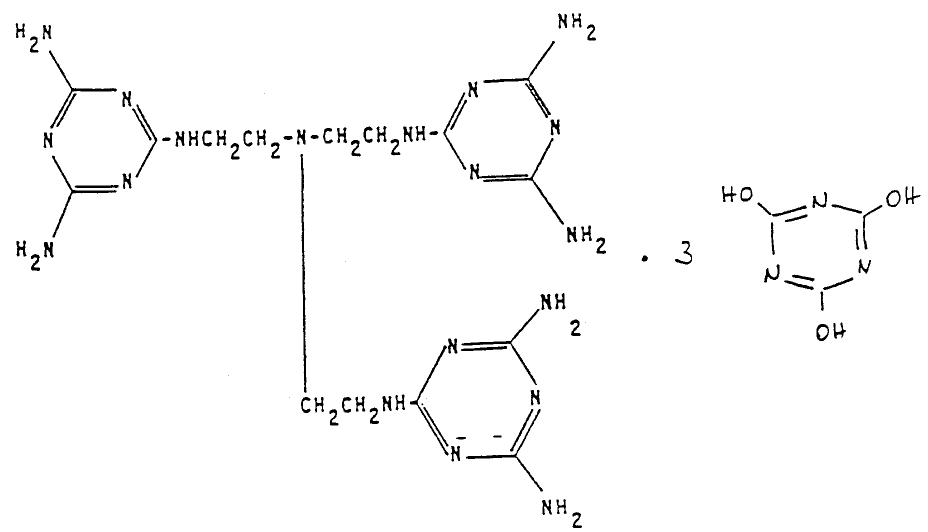
as a white crystalline powder having a m.p. greater than 300°.

### EXAMPLES 8-11

The cyanurate products of the general formula (I) reported in Table 2, all having a m.p. greater than 300°C, are synthesized by operating under the general procedure of Examples 1 to 7.

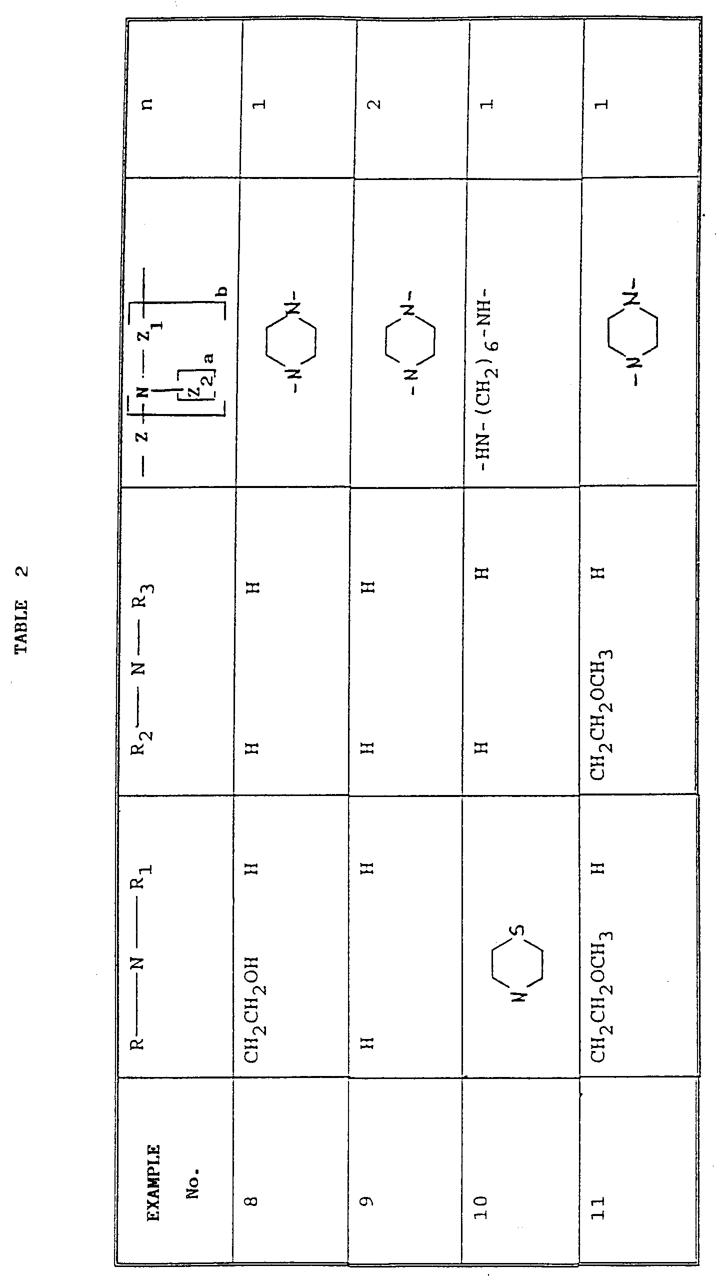

### TABLE 3

Table 3 shows the results of the thermogravimetric analysis (T.G.A.) of the cyanurates of Examples 1, 3, 6 and 10 of the present invention, compared to the derivatives of 2,4,6-triamino-1,3,5-triazine corresponding to these cyanurates. Since these derivatives are of general formula, they are indentified in Table 3 as simply (XIV).

The thermal stability of said products has been determined by evaluating the loss of weight with the increase in temperature. A DU PONT model 951-9900 thermobalance operated with an air flow of 5 liter/hour, at a heating gradient of 20°C/minute, with the temperature ranging from 20 to 600°C, and quantities of product of about 10 mg, was used to determine the weight loss with increasing temperature. The data of Table 3 demonstrate that the cyanurates of this invention have substantially greater thermostability than the 2,4,6-triamino-1,3,5-triazine derivatives from which they were prepared.

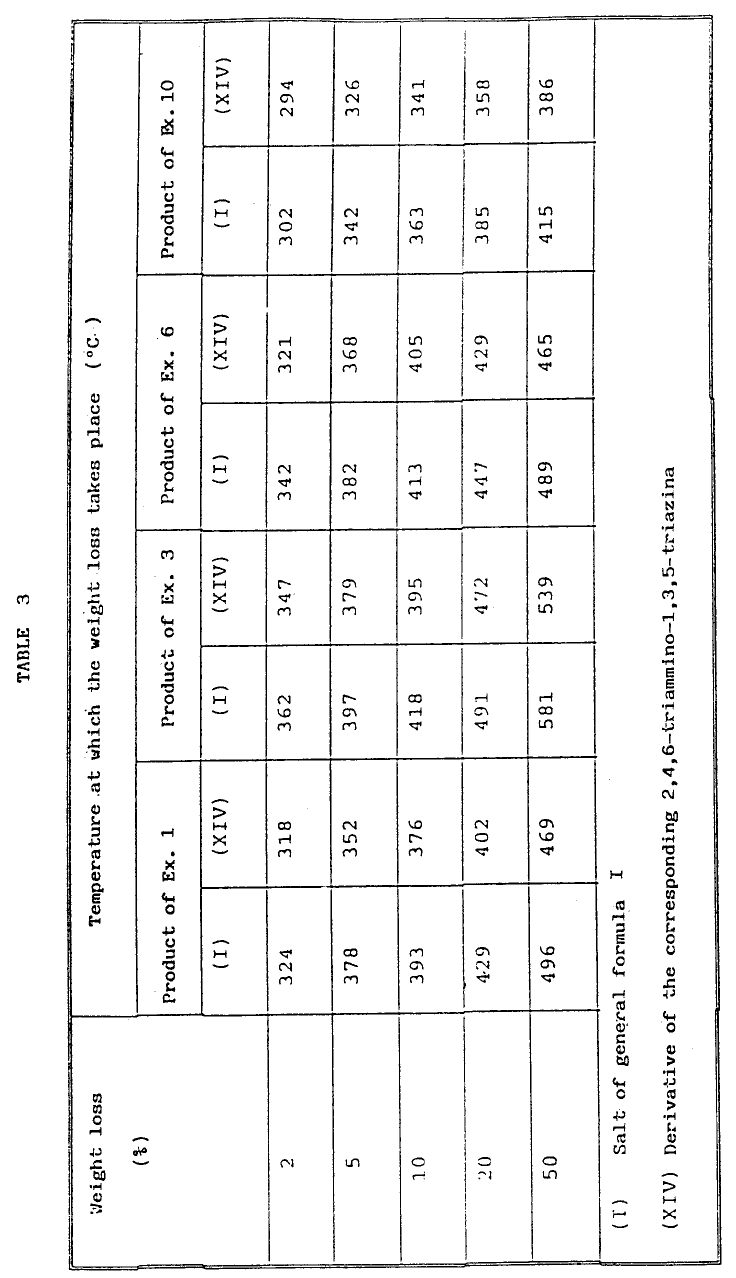

### EXAMPLES 12-33

Tables 4 and 5 report the data of the flame retardant testing of polymer compositions containing the salts of general formula (I) prepared according to the preceding Examples.

Specimens in the form of small 3 mm thick plates were prepared by molding in a MOORE plate press, for 7 minutes at a pressure of 40 atmospheres, blends of granular polymer and the additives.

Fire-retardation levels of the specimens were determined on the plates by measuring the oxygen index (L.O.I. according to ASTM D-2863/77) in a Stanton Redcroft apparatus, and by the Vertical Burning Test, by which the material being tested can be classified at one of three levels, 94 V-0, 94 V-1 and 94 V-2, according to UL 94 regulations (issued by Underwriters Laboratories in the USA).

Table 4 shows the values obtained by using an isotactic polypropylene in flake form, having a Melt Flow Rate (ASTM D-1238) equal to 12, and a fraction insoluble in boiling n-heptane equal to 96% by weight.

Table 5 shows the values obtained with a composition in which the polymer component is a low-density polyethylene in pellet form, having a Melt Flow Rate (ASTM D-1238) equal to 7; a polystyrene in pellet form containing 5% by weight of butadiene rubber, and having a Melt Flow Rate (ASTM D-1238) equal to 9; a thermoplastic polyester polyurethane (ESTANE 54600 produced by Goodrich) and polyether polyurethane (ESTANE 58300 produced by Goodrich) in pellet form, having a specific weight equal to 1.19 and 1.10 g/cm³ respectively; an elastomeric ethylene-propylene copolymer having a propylene content of 45% by weight; an acrylonitrile-butadiene-styrene terpolymer having a specific weight equal to 1.06 g/cm³, Melt Flow Rate (ASTM D-1238) equal to 1.6, and containing about 40% by weight of acrylonitrile and styrene, and 20% by weight of butadiene.

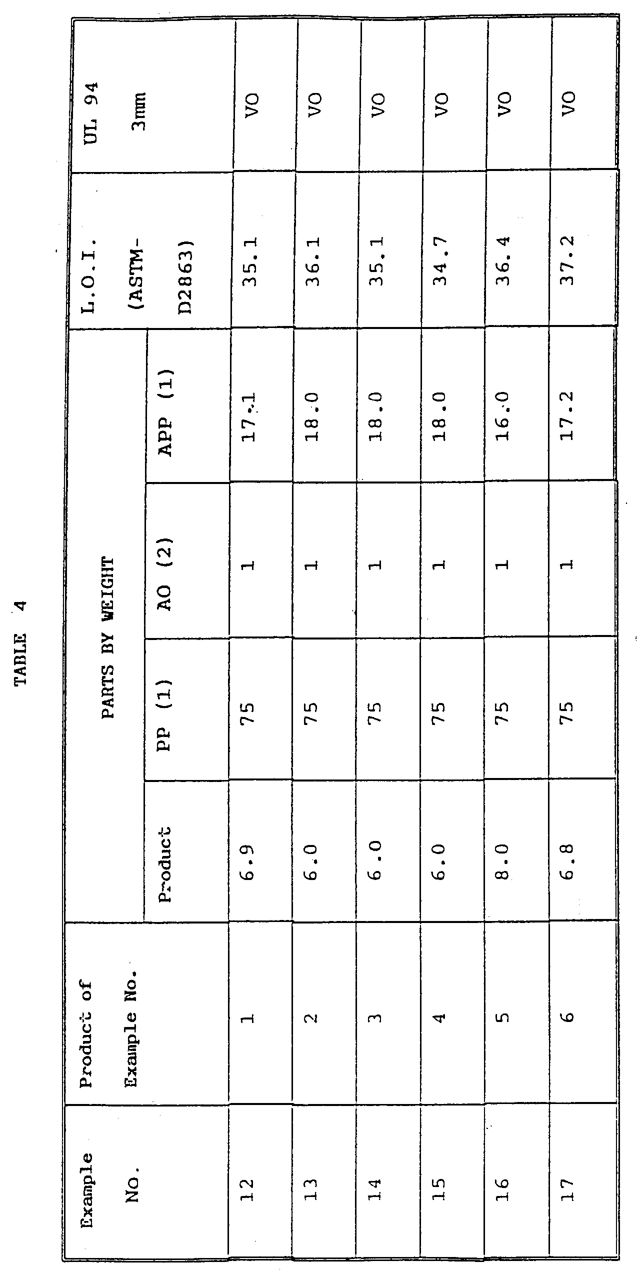

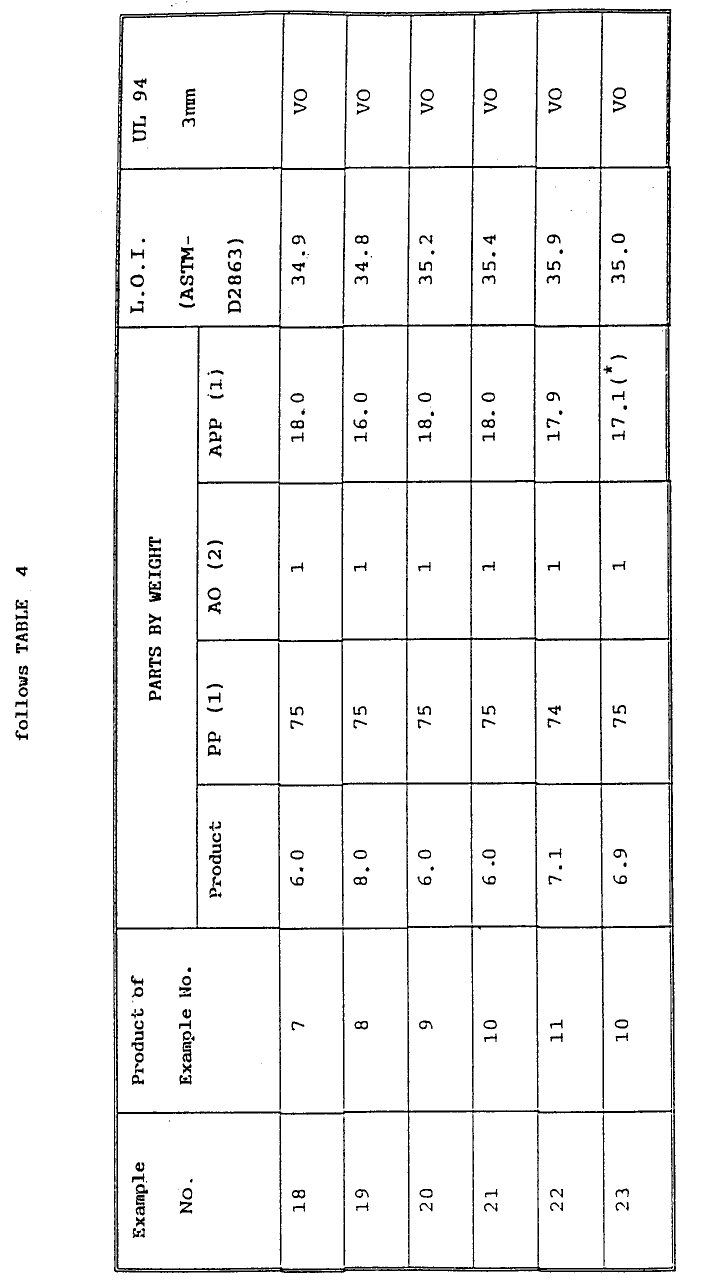

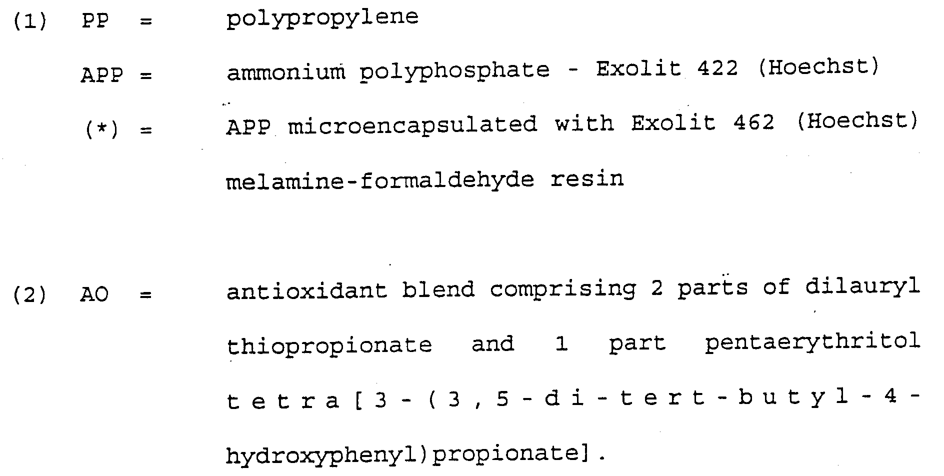

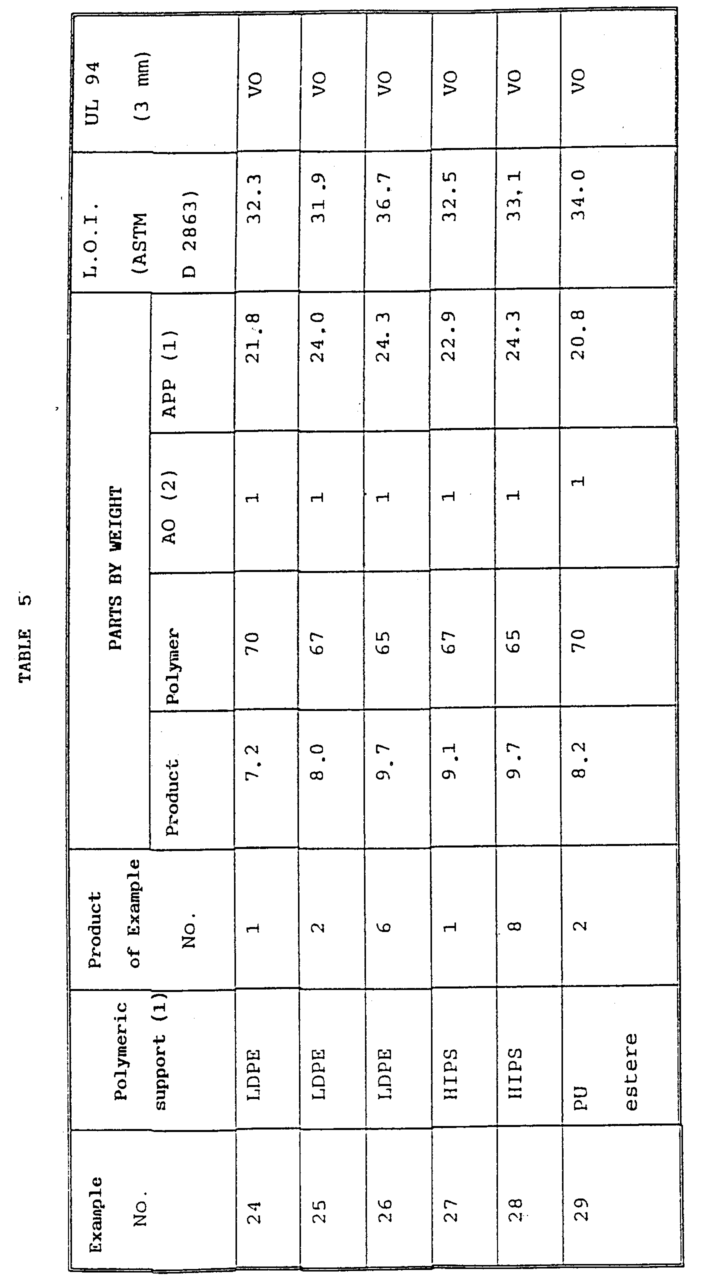

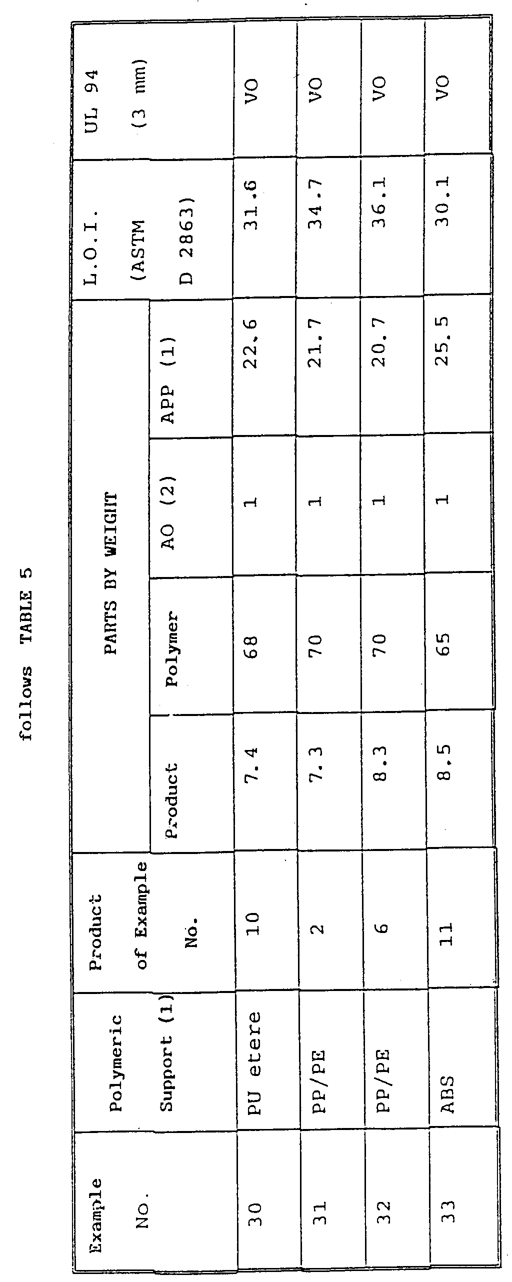

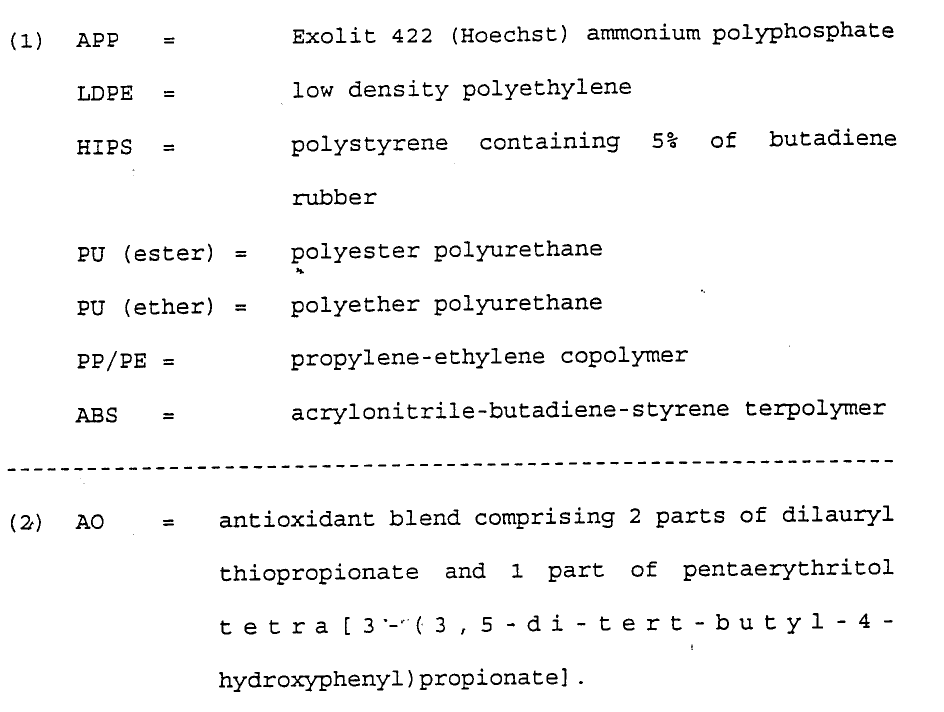

### COMPARATIVE EXAMPLE 34

Following the preparation and testing methods of Examples 12 to 23, but using the 2,4,6-triamino-1,3,5-triazine cyanurate (melamine cyanurate) as the nitrogenated compound, the composition specified below was prepared:

| | |
|---|---|
| Polypropylene: | 75 parts by weight |
| Antioxidant: | 1 part by weight |
| Ammonium polyphosphate: | 17.1 parts by weight |
| Melamine cyanurate: | 6.9 parts by weight |

Specimens of this composition were prepared and then subjected to the fire-retardant tests. The results were as follows:
L.O.I. = 22.4
UL94 (3 mm): Class B (the specimen ignites).

From the data of Tables 4 and 5 and the data of Comparative Example 34, it is clear that this invention provides fire retardant additives and polymer compositions having superior fire retardant properties.

## Claims

1. A flame-retardant polymer composition comprising:
a) 90 to 40 parts by weight of a thermoplastic or elastomeric polymer;
b) 6 to 33 parts by weight of at least one ammonium or amine phosphates or phosphonates or mixtures thereof;
c) 4 to 27 parts by weight of at least one cyanuric acid salt of a derivative of 2,4,6-triamino-1,3,5-triazine containing two or more triazine rings, said salts having the general formula (I): 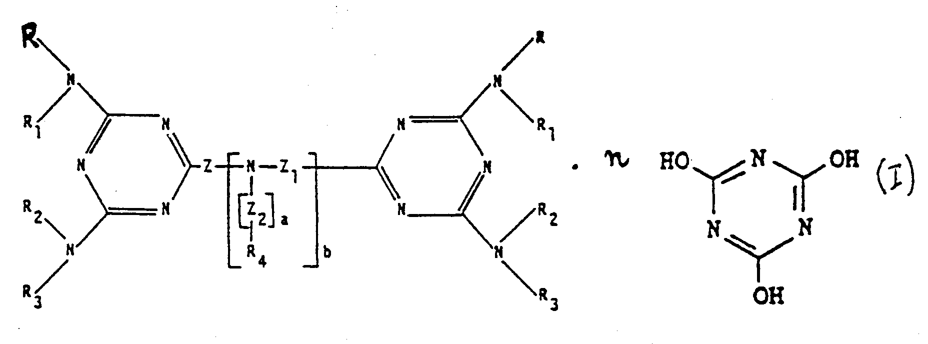 wherein:
n is an integer from 1 to 7;
the radicals from R to R₃ are the same or different, and also can be different from one triazine ring to another triaxine ring, and are:
H; C₁-C₁₈ alkyl, C₂-C₈ alkenyl; C₆-C₁₆ cycloalkyl or alkylcycloalkyl, optionally substituted with a C₁-C₄ hydroxyl or hydroxyalkyl function; 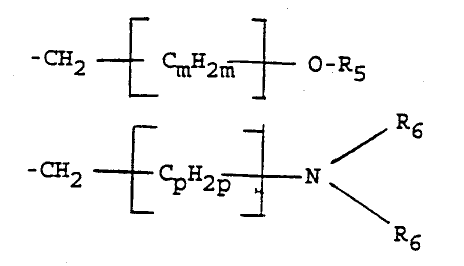 wherein:
m = an integer from 1 to 7;
p = an integer from 1 to 5,
R₅ = H; C₁-C₈ alkyl, C₂-C₆ alkenyl; ⁅C_{q}H_{2q}⁆O-R₇ wherein q is an integer from 1 to 4 and R₇ is H, C₁-C₄ alkyl, C₆-C₁₂ cycloalkyl or C₆-C₁₂ alkylcycloalkyl;
the R₆ radicals can be the same or different and are:
H; C₁-C₈ alkyl; C₂-C₆ alkenyl; C₆-C₁₂ cycloalkyl; C₆-C₁₂ alkylcycloalkyl; C₁-C₄ hydroxyalkyl;
or the group: 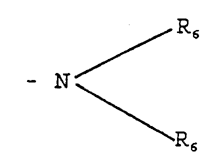
is substituted with a heterocyclic radical bonded to the alkyl chain by the nitrogen atom and optionally containing another heteroatom;
or in the general formula (I) at least one of the groups: 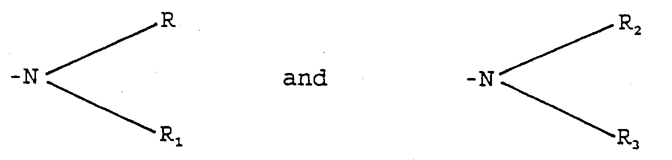 is substituted with a heterocyclic radical bonded to the triazine ring by the nitrogen atom, and optionally containing another heteroatom;
a is O or 1;
b is O or an integer from 1 to 5;
R₄ is H or: 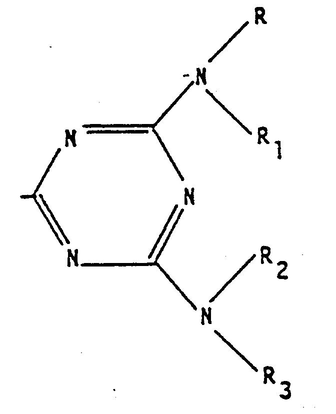 and its meaning can vary inside each repetitive unit;
when b is O, Z is a bivalent radical of one of the following formulas II-XI: 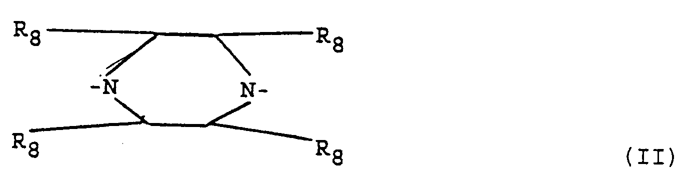 wherein the R₈ radicals, which can be the same or different, are H or C₁-C₄ alkyl; 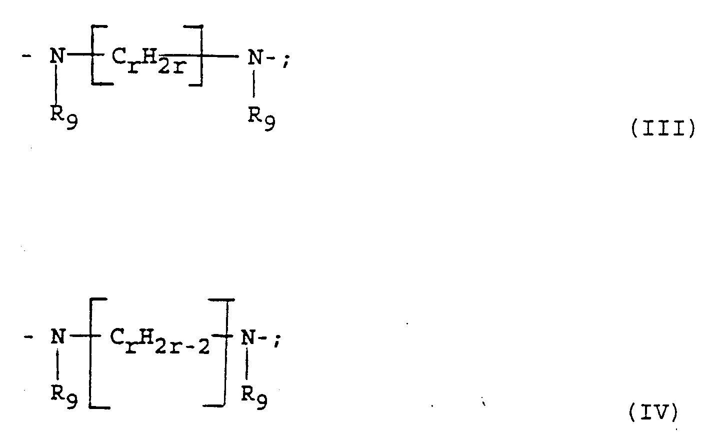 wherein r is an integer from 2 to 14; R₉ is H; C₁-C₄ alkyl; C₂-C₆ alkenyl; C₁-C₄ hydroxyalkyl; 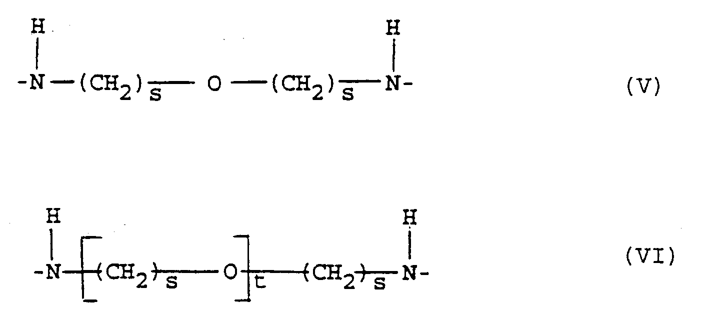 wherein s is an integer from 2 to 5, and t is an integer from 1 to 3; 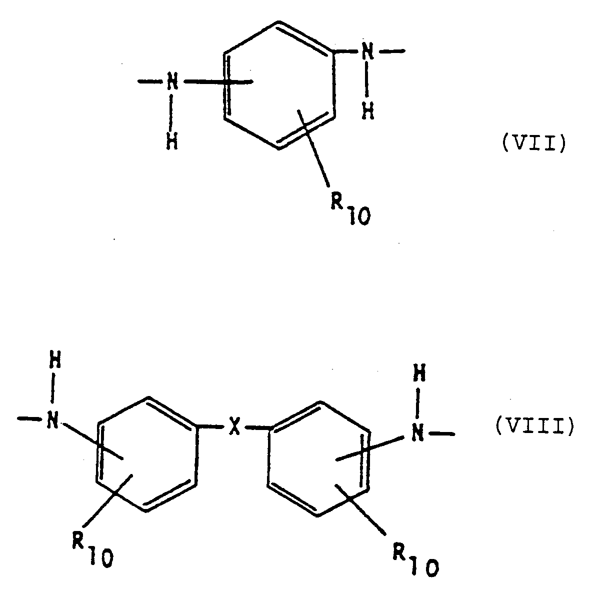 wherein:
X is a C-C direct bond, O, S, S-S, SO, SO₂, NH, NHSO₂, NHCO, N=N, or CH₂;
R₁₀ is H, hydroxyl, C₁-C₄ alkyl or C₁-C₄ alkoxyl; 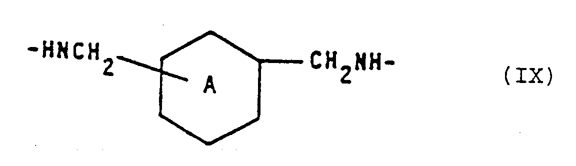 where A is a saturated or unsaturated cyclic ring. 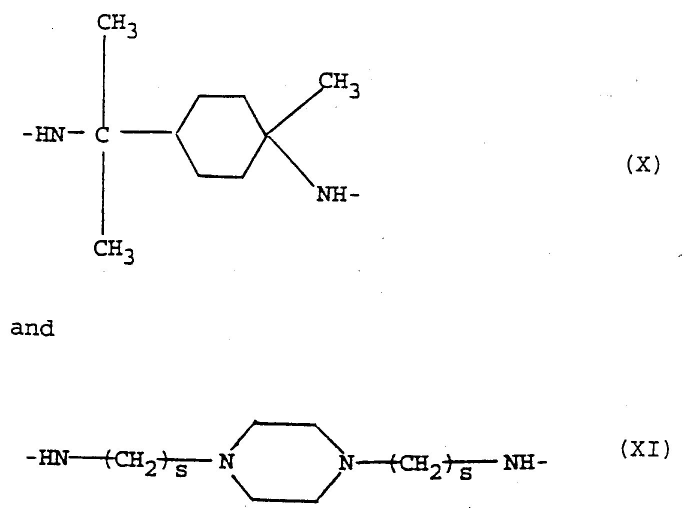 wherein s is as defined above; and
when b is an integer from 1 to 5, the group: 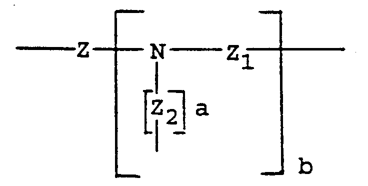 has one of the following formulas XII or XIII: 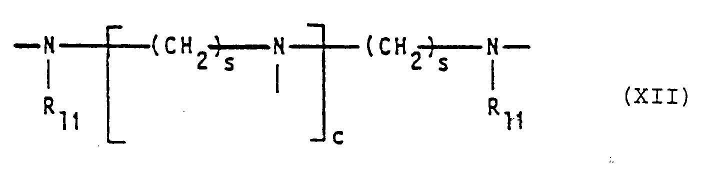 wherein:
R₁₁ is H or C₁-C₄ alkyl;
c is an integer from 1 to 5; and
the s can be the same or different and are as defined above; 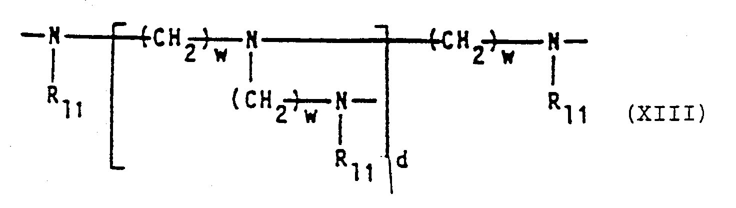 wherein:
R₁₁ is as defined above;
w is an integer from 2 to 4;
d is 1 or 2.

2. The flame-retardant polymer composition of claim 1, wherein at least one of the groups: 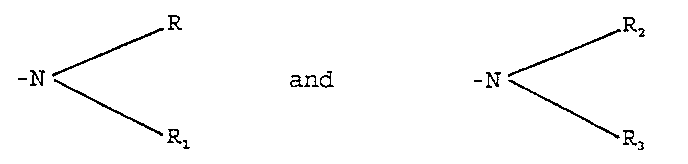 in the general formula (I) is substituted with a heterocyclic radical bonded to the triazine ring by the nitrogen atom and selected from the group consisting of aziridinyl; pyrrolidinyl; piperidinyl; morpholinyl; thiomorpholinyl; piperazinyl; 4-methylpiperazinyl; 4-ethylpiperazinyl; 2-methylpiperazinyl; 2,5-dimethylpiperazinyl; 2,3,5,6-tetramethylpiperazinyl; 2,2,5,5-tetramethylpiperazinyl; 2-ethylpiperazinyl; 2,5-diethylpiperazinyl.

3. The flame-retardant polymer composition of claim 1, wherein at least one of the radicals from R to R₃ in general formula (I) is substituted with a group: -CH₂⁅CₘH₂ₘ⁆O-R₅; wherein:
m is an integer from 1 to 3 and R₅ is H or a C₁-C₄ alkyl.

4. The flame-retardant polymer composition of claim 1, wherein the group: 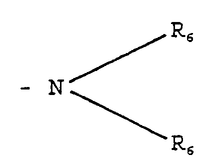 in general formula (I) is substituted with a heterocyclic radical bonded to the alkyl chain by the nitrogen atom and selected from the group consisting of the group consisting of:aziridinyl; pyrrolidinyl; piperidinyl; morpholinyl; thiomorpholinyl; piperazinyl; 4-methylpiperazinyl; 4-ethylpiperazinyl.

5. The flame-retardant polymer composition of claim 1, wherein at least one of the groups: 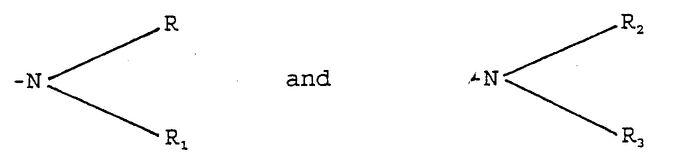 in general formula (I) is an NH₂ group.

6. The flame-retardant polymer composition of claim 1, wherein the ammonium phosphate or phosphates (b) have the general formula (NH₄)ₙ₊₂ Pₙ O₃ₙ₊₁ wherein n is an integer equal to or greater than 2.

7. The flame-retardant polymer composition of claim 1, wherein the ammonium phosphate or phosphates (b) have the general formula (NH₄PO₃)ₙ wherein n is an integer from 50 to 500.

8. The flame-retardant polymer composition of claim 1, wherein the amine phosphate or phosphates (b) are selected from the group consisting of dimethylammonium phosphate, diethylammonium phosphate, ethylenediamine phosphate, melamine ortho- or pyrophosphate.

9. The flame-retardant polymer composition of claim 1, wherein the ammonium phosphonate or phosphonates (b) are mono- or polysubstituted and are selected from the mono- and polyphosphonic acid derivatives.

10. The flame-retardant polymer composition of claim 1, wherein polymer (a) is selected from the group consisting of polymers and copolymers of olefins of general formula R-CH=CH₂, wherein R is a hydrogen atom or C₁-C₈ alkyl or C₆-C₈ aryl; acrylonitrile/butadiene/styrene (ABS) copolymers; styrene/acrylonitrile (SAN) copolymers; polyurethane; polyethylene terephtalate; polybutylene terephtalate; polyamides.

11. The flame-retardant polymer composition of claim 10, wherein the olefin polymers and copolymers are selected from the group consisting of:
1. polypropylene with isotactic index greater than 80;
2. HDPE, LLDPE, LDPE polyethylene;
3. crystalline propylene copolymers with minor proportions of ethylene, other C₄-C₈ α-olefins, or mixtures of ethylene and the C₄-C₈ α-olefins;
4. heterophasic olefin polymer compositions comprising (A) a crystalline homopolymer fraction of propylene or a copolymer of item (3), and (B) a copolymer fraction comprising an elastomeric copolymer of ethylene with a C₃-C₈ α-olefin, and optionally a minor proportion of a diene; and
5. elastomeric copolymers of ethylene, C₃-C₈ α-olefins and optionally a minor proportion of a diene.

12. A molded article obtained from the composition of claim 1.

13. A cyanuric acid salt of a derivative of 2,4,6-triamino-1,3,5-triazine containing two or more triazine rings, said salt having the general formula (I): 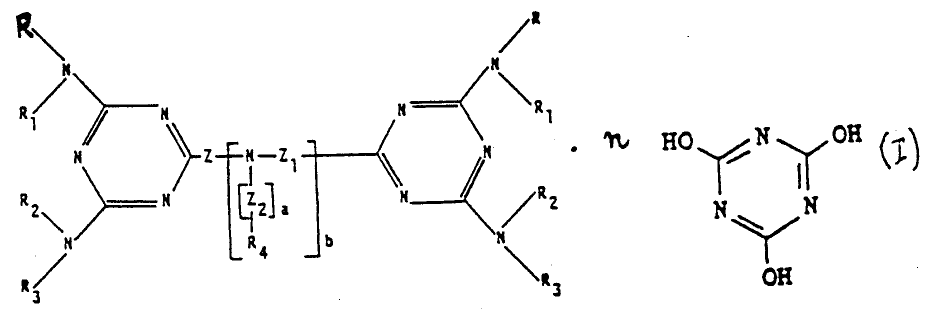 wherein:
n is an integer from 1 to 7;
the radicals from R to R₃ are the same or different, and also can be different from one triazine ring to another triazine ring, and are:
H; C₁-C₁₈ alkyl, C₂-C₈ alkenyl; C₆-C₁₆ cycloalkyl or alkylcycloalkyl, optionally substituted with a C₁-C₄ hydroxyl or hydroxyalkyl function; 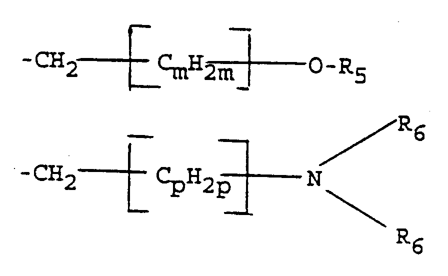 wherein:
m = an integer from 1 to 7;
p = an integer from 1 to 5,
R₅ = H; C₁-C₈ alkyl; C₂-C₆ alkenyl; ⁅C_{q}H_{2q}⁆O-R₇ wherein q is an integer from 1 to 4 and R₇ is H or C₁-C₄ alkyl, C₆-C₁₂ cycloalkyl or C₆-C₁₂ alkylcycloalkyl;
the R₆ radicals can be the same or different and are: H; C₁-C₈ alkyl; C₂-C₆ alkenyl; C₆-C₁₂ cycloalkyl or C₆-C₁₂ alkylcycloalkyl; C₁-C₄ hydroxyalkyl;
or the group: 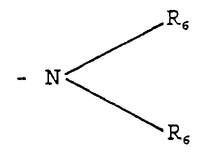 is substituted with a heterocyclic radical bonded to the alkyl chain by the nitrogen atom and optionally containing another heteroatom;
or in the general formula (I) at least one of the groups: 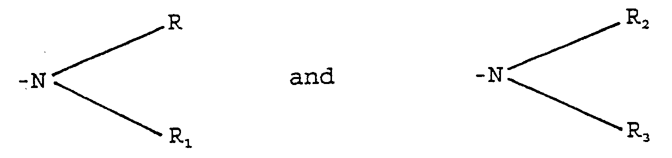 is substituted with a heterocyclic radical bonded to the triazine ring by the nitrogen atom, and optionally containing another heteroatom;
a is O or 1;
b is O or an integer from 1 to 5;
R₄ is H or: 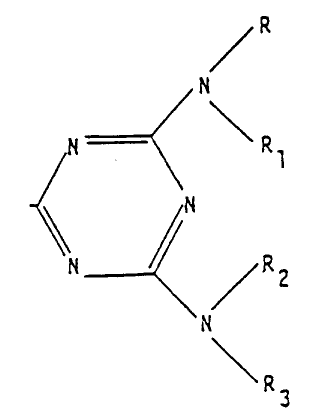 and its meaning can vary inside each repetitive unit; when b is O, Z is a bivalent radical of one of the following formulas II-XI: 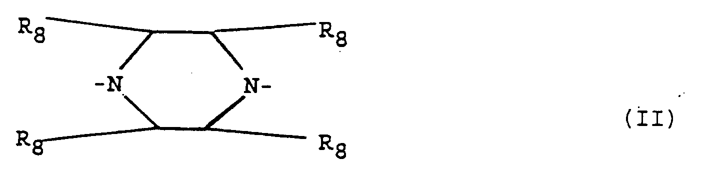 wherein the R₈ radicals, which can be the same or different, are H or C₁-C₄ alkyl; 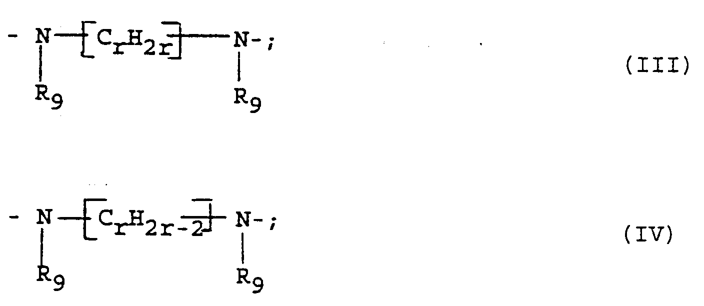 wherein r is an integer from 2 to 14; R₉ is H; C₁-C₄ alkyl; C₂-C₆ alkenyl; C₁-C₄ hydroxyalkyl;
C₁-C₄; 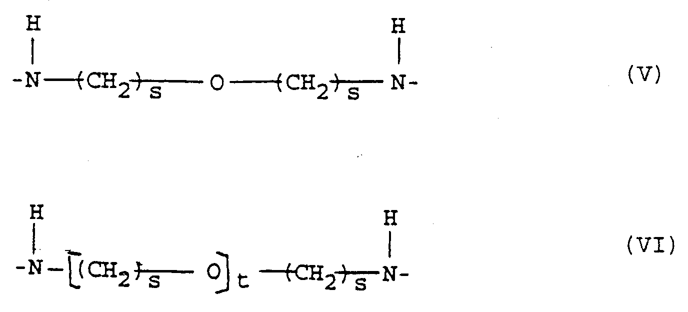 wherein s is an integer from 2 to 5, and t is an integer from 1 to 3; 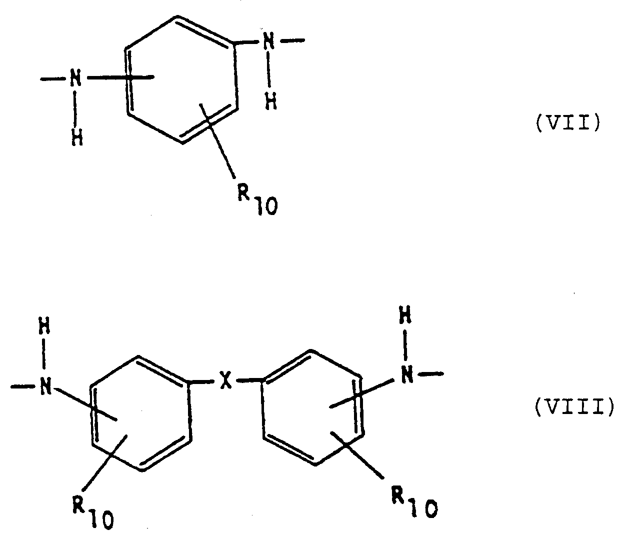 wherein:
X is a C-C direct bond, O, S, S-S, SO, SO₂, NH, NHSO₂, NHCO, N=N, or CH₂;
R₁₀ is H, hydroxyl, C₁-C₄ alkyl or C₁-C₄ alkoxyl; 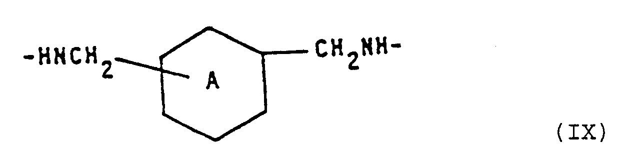 wherein A is a saturated or unsaturated cyclic ring; 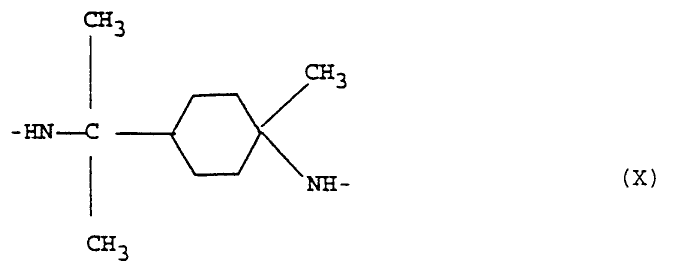 and 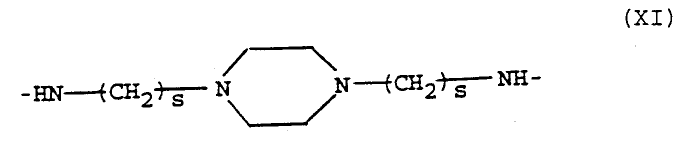 wherein s is as defined above; and
when b is an integer from 1 to 5, the group: 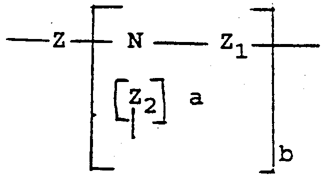 has one of the following formulas XII or XIII: 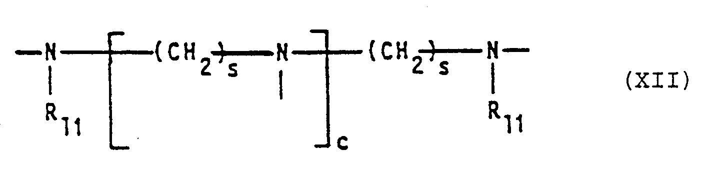 wherein:
R₁₁ is H or C₁-C₄ alkyl;
c is an integer from 1 to 5; and
the s can be the same or different and are as defined above; 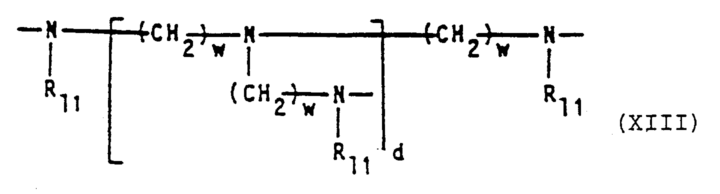 wherein:
R₁₁ is as defined above;
w is an integer from 2 to 4;
d is 1 or 2;
provided that when the radicals from R to R₃ are hydrogen, Z is a bivalent (II) or (XI) radical, or a polyvalent (XIII) radical.

14. The salt of claim 13, wherein at least one of the groups: 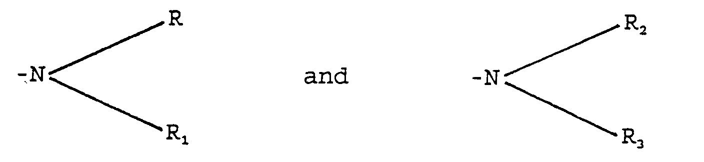 in general formula (I) is substituted with a heterocyclic radical bonded to the triazine ring by the nitrogen atom and selected from the group consisting of: aziridinyl; pyrrolidinyl; piperidinyl; morpholinyl; thiomorpholinyl; piperazinyl; 4-methylpiperazinyl; 4-ethylpiperazinyl; 2-methylpiperazinyl; 2,5-dimethylpiperazinyl; 2,3,5,6-tetramethylpiperazinyl; 2,2,5,5-tetramethylpiperazinyl; 2-ethylpiperazinyl; 2,5-diethylpiperazinyl.

15. The salt of claim 13, wherein the group: 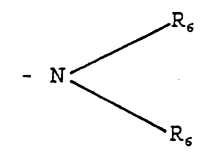 is substituted with a heterocyclic radical bonded to the alkyl chain by the nitrogen atom and selected from the group consisting of:aziridinyl; pyrrolidinyl; piperidinyl; morpholinyl; thiomorpholinyl; piperazinyl; 4-methylpiperazinyl; 4-ethylpiperazinyl.

16. A process for the preparation of a salt of general formula (I) of claim 13, comprising reacting a derivative of 2,4,6-triamino-1,3,5-triazine of general formula (XIV): 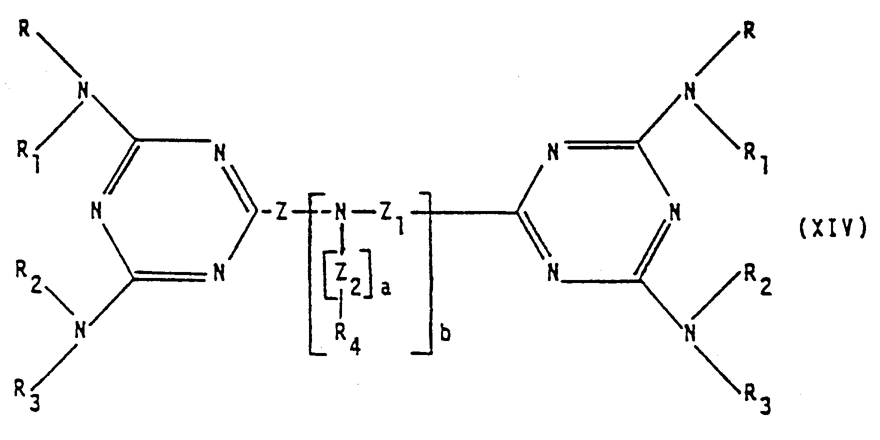 wherein:
the substituents from R, R₁, R₂, R₃, R₄ and the radical: 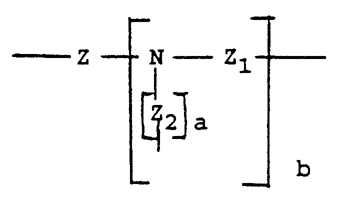 have the meaning defined in claim 13, with cyanuric acid in amount from 1 to 7 moles per mole of the derivative of formula (XIV).

17. The process of claim 16, wherein the reaction between the derivative of general formula (XIV) and cyanuric acid is carried out in the presence of a solvent at a temperature from 0°C to the boiling point of the solvent.
